# EUROPEAN PATENT SPECIFICATION

(11) **EP 3 860 599 B1**
(45) Date of publication and mention of the grant of the patent: **15.05.2024**
(21) Application number: 19787167.6
(22) Date of filing: 03.10.2019
(51) Int. Cl.: A61K 31/4375, A61P 21/00

(54) **(+)-ALPHA-DIHYDROTETRABENAZINE DOSAGE REGIMEN FOR TREATING MOVEMENT DISORDERS**
(+)-ALPHA-DIHYDROTETRABENAZIN-DOSIERUNGSSCHEMA ZUR BEHANDLUNG VON BEWEGUNGSSTÖRUNGEN
RÉGIME POSOLOGIQUE DE (+)-ALPHA-DIHYDROTÉTRABÉNAZINE DESTINÉ AU TRAITEMENT DE TROUBLES DU MOUVEMENT

(30) Priority: 04.10.2018 GB 201816192
(43) Date of publication of application: 11.08.2021
(73) Proprietor: Adeptio Pharmaceuticals Limited, London SW1Y 4LB (GB)
(72) Inventor: DUFFIELD, Andrew John, London SW1Y 4LB (GB); PANDYA, Anant, London SW1Y 4LB (GB)
(74) Representative: Hutchins, Michael Richard
(86) International application number: PCT/EP2019/076800
(87) International publication number: WO 2020/070236

(56) References cited:
- WO-A1-2015/171802
- WO-A1-2018/178233
- WO-A1-2018/178251
- WO-A2-2015/120110

## Description

### Background of the Invention

Movement disorders can generally be classified into two categories: hyperkinetic movement disorders and hypokinetic movement disorders. Hyperkinetic movement disorders are caused by an increase in muscular activity and can cause abnormal and/or excessive movements, including tremors, dystonia, chorea, tics, myoclonus and stereotypies.

Hyperkinetic movement disorders often are often psychological in nature and arise through improper regulation of amine neurotransmitters in the basal ganglia.

A particular hyperkinetic movement disorder is Tourette's syndrome, which is an inherited neurological condition characterised by multiple physical and vocal tics. The tics are usually repetitive, but random, physical movements or vocal noises. The vocal tics can be of various forms and include repeating one's own words, the words of others or other sounds. Onset usually occurs in children and continues through to adolescence and adulthood.

While the tics associated with Tourette's syndrome are temporarily suppressible, those affected can usually only suppress their tics for limited time periods. There is yet to be an effective treatment to cover all types of tics in all patients, but certain medicaments for tic suppression have been developed.

It is known that dopamine receptor antagonists display an ability to suppress tics in Tourette's syndrome patients and a number of dopamine receptor antagonists are currently used in the suppression of Tourette's tics, such as fluphenazine, risperidone, haloperidol and pimozide.

Type 2 vesicular monoamine transporter (VMAT2) is a membrane protein responsible for the transportation of monoamine neurotransmitters, such as dopamine, serotonin and histamine, from cellular cytosol into synaptic vesicles. Inhibition of this protein hinders presynaptic neurons from releasing dopamine, resulting in a depletion of dopamine levels in the brain.

VMAT2 inhibitors can be used to treat the symptoms of Tourette's syndrome.

Tetrabenazine (Chemical name: 1,3,4,6,7,11b-hexahydro-9,10-dimethoxy-3-(2-methylpropyl)-2H-benzo(a)quinolizin-2-one) has been in use as a pharmaceutical drug since the late 1950s. Initially used as an anti-psychotic, tetrabenazine is currently used for treating hyperkinetic movement disorders such as Huntington's disease, hemiballismus, senile chorea, tic, tardive dyskinesia and Tourette's syndrome, see for example Jankovic et al., Am. J. Psychiatry. (1999) Aug; 156(8):1279-81 and Jankovic et al., Neurology (1997) Feb; 48(2):358-62.

The primary pharmacological action of tetrabenazine is to reduce the supply of monoamines (e.g. dopamine, serotonin, and norepinephrine) in the central nervous system by inhibiting the human vesicular monoamine transporter isoform 2 (hVMAT2). The drug also blocks post-synaptic dopamine receptors.

The central effects of tetrabenazine closely resemble those of reserpine, but it differs from reserpine in that it lacks activity at the VMAT1 transporter. The lack of activity at the VMA T1 transporter means that tetrabenazine has less peripheral activity than reserpine and consequently does not produce VMAT1-related side effects such as hypotension.

Tetrabenazine is an effective and safe drug for the treatment of a variety of hyperkinetic movement disorders and, in contrast to typical neuroleptics, has not been demonstrated to cause tardive dyskinesia. Nevertheless, tetrabenazine does exhibit a number of dose-related side effects including causing depression, parkinsonism, drowsiness, nervousness or anxiety, insomnia and, in rare cases, neuroleptic malignant syndrome, see for example the introductory section of WO2016/127133 (Neurocrine Biosciences).

The chemical structure of tetrabenazine is as shown below.

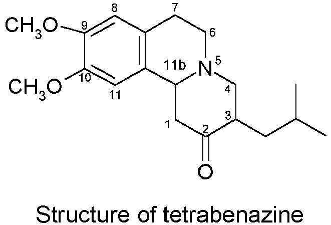

The compound has chiral centres at the 3 and 11b carbon atoms and hence can, theoretically, exist in a total of four isomeric forms, as shown below.

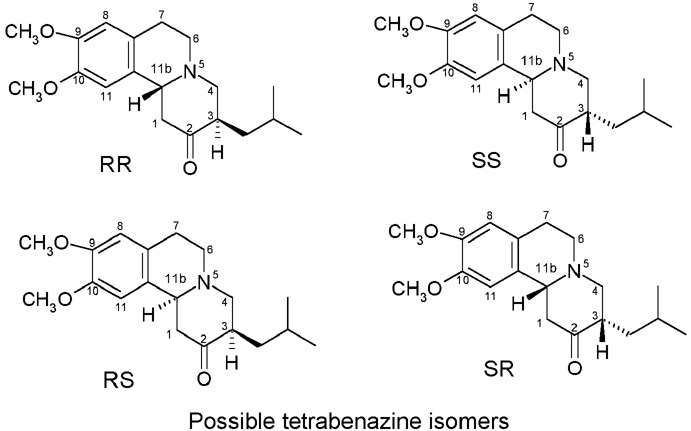

The stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In this patent application, the designations "R" or "S" are given in the order of the position numbers of the carbon atoms. Thus, for example, *RS* is a shorthand notation for 3*R*,11b*S*. Similarly, when three chiral centres are present, as in the dihydrotetrabenazines described below, the designations "*R*" or "*S*" are listed in the order of the carbon atoms 2, 3 and 11b. Thus, the 2*R*,3S, *11bS* isomer is referred to in short hand form as *RSS* and so on.

Commercially available tetrabenazine is a racemic mixture of the *RR* and *SS* isomers and it would appear that the *RR* and *SS* isomers are the most thermodynamically stable isomers.

Tetrabenazine has somewhat poor and variable bioavailability. It is extensively metabolised by first-pass metabolism, and little or no unchanged tetrabenazine is typically detected in the urine. It is known that at least some of the metabolites of tetrabenazine are dihydrotetrabenazines formed by reduction of the 2-keto group in tetrabenazine.

Dihydrotetrabenazine (Chemical name: 2-hydroxy-3-(2-methylpropyl)-1,3,4,6,7,11 b-hexahydro-9,10-dimethoxy-benzo(a)quinolizine) has three chiral centres and can therefore exist in any of the following eight optical isomeric forms:

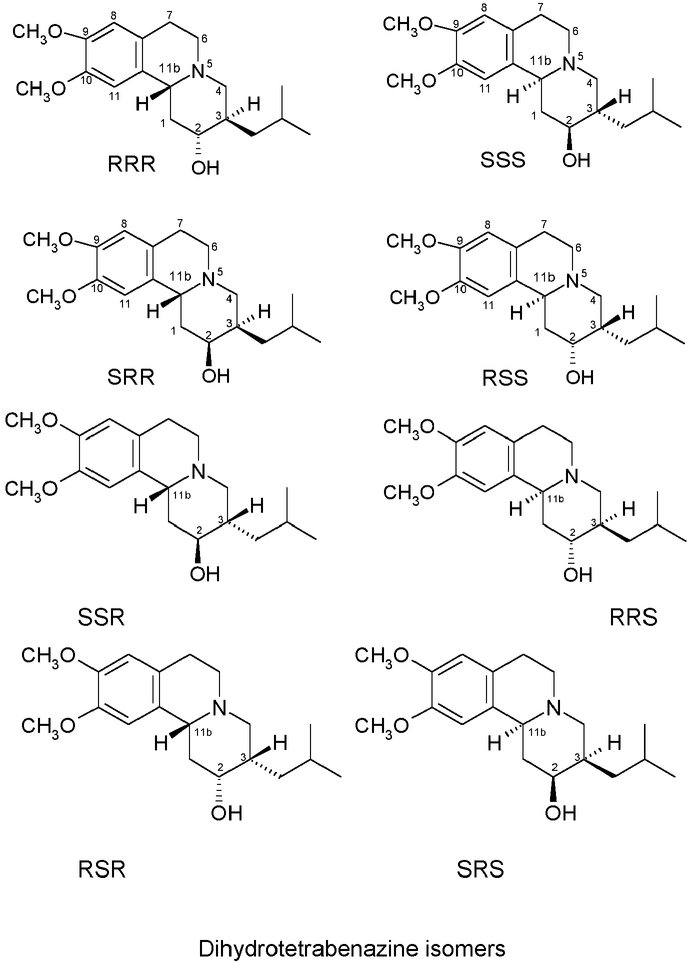

The synthesis and characterisation of all eight dihydrotetrabenazine isomers is described by Sun et al. (Eur. J. Med. Chem. (2011), 1841-1848).

Of the eight dihydrotetrabenazine isomers, four isomers are derived from the more stable RR and SS isomers of the parent tetrabenazine, namely the RRR, SSS, SRR and RSS isomers.

The RRR and SSS isomers are commonly referred to as "alpha (α)" dihydrotetrabenazines and can be referred to individually as (+)-α-dihydrotetrabenazine and (-)-α-dihydrotetrabenazine respectively. The alpha isomers are characterised by a *trans* relative orientation of the hydroxyl and 2-methylpropyl substituents at the 2- and 3-positions - see for example, Kilbourn et al., Chirality, 9:59-62 (1997) and Brossi et al., Helv. Chim. Acta., vol. XLI, No. 193, pp1793-1806 (1958).

The SRR and RSS isomers are commonly referred to as "beta (β)" isomers and can be referred to individually as (+)-β-dihydrotetrabenazine and (-)-β-dihydrotetrabenazine respectively. The beta isomers are characterised by a *cis* relative orientation of the hydroxyl and 2-methylpropyl substituents at the 2- and 3-positions.

Although dihydrotetrabenazine is believed to be primarily responsible for the activity of the drug, there have been no studies published to date that contain evidence demonstrating which of the various stereoisomers of dihydrotetrabenazine is responsible for its biological activity. More specifically, there have been no published studies demonstrating which of the stereoisomers is responsible for the ability of tetrabenazine to treat movement disorders such as Tourette's syndrome.

Schwartz et al. (Biochem. Pharmacol. (1966), 15: 645-655) describes metabolic studies of tetrabenazine carried out in rabbits, dogs and humans. Schwartz *et al.* identified nine metabolites, five of which were unconjugated and the other four of which were conjugated with glucuronic acid. The five unconjugated metabolites were the alpha- and beta-dihydrotetrabenazines, their two oxidised analogues in which a hydroxyl group has been introduced into the 2-methylpropyl side chain, and oxidised tetrabenazine in which a hydroxyl group has been introduced into the 2-methylpropyl side chain. The four conjugated metabolites were all compounds in which the 9-methoxy group had been demethylated to give a 9-hydroxy compound. The chirality of the various metabolites was not studied and, in particular, there was no disclosure of the chirality of the individual α- and β-isomers.

Scherman et al., (Mol. Pharmacol. (1987), 33, 72-77 describes the stereospecificity of VMAT2 binding between racemic α- and β- dihydrotetrabenazine. They reported that α-dihydrotetrabenazine had a 3- to 4-fold higher affinity for the Chromaffin Granule Monoamine Transporter than the β-isomer, when studied in vitro. However, Scherman *et al.* does not disclose the resolution or testing of the individual enantiomers of the α- and β-dihydrotetrabenazines.

Mehvar et al. (J. Pharm. Sci. (1987), 76(6), 461-465) reported a study of the concentrations of tetrabenazine and dihydrotetrabenazine in the brains of rats following administration of either tetrabenazine or dihydrotetrabenazine. The study showed that despite its greater polarity, dihydrotetrabenazine was able to cross the blood-brain barrier. However, the stereochemistry of the dihydrotetrabenazine was not disclosed.

Mehvar et al. (Drug Metabolism and Disposition (1987), 15:2, 250-255) describes studies of the pharmacokinetics of tetrabenazine and dihydrotetrabenazine following administration of tetrabenazine to four patients affected by tardive dyskinesia. Oral administration of tetrabenazine resulted in low plasma concentrations of tetrabenazine but relatively high concentrations of dihydrotetrabenazine. However, the stereochemistry of the dihydrotetrabenazine formed *in vivo* was not reported.

Roberts et al. (Eur. J. Clin. Pharmacol. (1986), 29: 703-708) describes the pharmacokinetics of tetrabenazine and its hydroxy-metabolite in patients treated for involuntary movement disorders. Roberts *et al.* reported that tetrabenazine was extensively metabolised after oral administration resulting in very low plasma concentrations of tetrabenazine but much higher concentrations of a hydroxy-metabolite. Although they did not describe the identity of the hydroxy-metabolites, they suggested that the high plasma concentrations of the hydroxy-metabolites may be therapeutically important (since the metabolites were known to be pharmacologically active) and that, in view of the disclosure in Schwartz *et al.* (*idem*), the combination of *cis* and *trans* isomers (i.e. beta and alpha isomers) could be more therapeutically important than the parent drug.

Michael Kilbourn and collaborators at the University of Michigan Medical School have published a number of studies relating to the various isomers of dihydrotetrabenazines. Thus, in Med. Chem. Res. (1994), 5:113-126, Kilbourn *et* al. describe the use (+/-)-α-[11C]-dihydrotetrabenazine as *in vivo* imaging agents for VMAT2 binding studies.

In Eur. J. Pharmacol (1995) 278, 249-252, Kilbourn *et al.* reported competition binding studies using [³H]-tetrabenazine to study the *in vitro* binding affinity of (+)-, (-)-, and (+/-)-α-DHTBZ. The binding assays gave Ki values of 0.97 nM for (+)-α-dihydrotetrabenazine and 2.2 µM for (-)-α-dihydrotetrabenazine, thereby showing that the (+) alpha isomer has much greater binding affinity for the VMAT2 receptor than the (-) alpha isomer. However, no studies were reported, or conclusions drawn, as to the usefulness of either isomer in the treatment of movement disorders such as Tourette's syndrome.

In Chirality (1997) 9:59-62, Kilbourn *et al.* described studies aimed at identifying the absolute configuration of (+)-α-dihydrotetrabenazine from which they concluded that it has the 2R, 3R, 11bR configuration shown above. They also referred to the Schwartz *et al.* and Mehvar *et al.* articles discussed above as indicating that the α- and β-dihydrotetrabenazines are likely to be the pharmacologically active agents in the human brain but they drew no explicit conclusions as to the precise stereochemical identities of the active metabolites of tetrabenazine.

In Synapse (2002), 43:188-194, Kilbourn *et al.* described the use of (+)-α-[¹¹C]-dihydrotetrabenazine as an agent used to measure specific *in vivo* binding of the VMAT receptor, in "infusion to equilibrium methods". They found that (-)-α-[¹¹C]-dihydrotetrabenazine produced a uniform brain distribution, consistent with the earlier observations that this enantiomer has a low VMAT affinity.

Sun *et al.* (*idem*) investigated the VMAT2 binding affinities of all eight dihydrotetrabenazine isomers. They found that all of the dextrorotatory enantiomers exhibited dramatically more potent VMAT2 binding activity than their corresponding laevorotatory enantiomers with the most active (+)-α-isomer being found to be the most active. However, Sun *et al.* did not carry out any investigations into the relative efficacies of the individual isomers in treating movement disorders such as Tourette's syndrome.

WO2015/120110 (Auspex) describes extended-release formulations that can contain any of a wide variety of different pharmacological agents, including tetrabenazine and dihydrotetrabenazine. WO2015/120110 discloses in general terms that the formulations may comprise between 5mg and 30mg of tetrabenazine or dihydrotetrabenazine and more specifically discloses extended-release formulations comprising tetrabenazine or dihydrotetrabenazine in amounts of 7.5 mg, 12.5 mg, 15 mg, 25 mg, 30 mg and 50 mg. However, there is no specific disclosure of formulations containing such amounts of (+)-α-dihydrotetrabenazine. Furthermore, there are no worked examples of any dihydrotetrabenazine formulations; but only formulations containing tetrabenazine.

WO 2006/053067 (Prestwick) describes the use of combinations of amantadine and a tetrabenazine compound (which can be tetrabenazine or dihydrotetrabenazine) for treating hyperkinetic movement disorders. WO 2006/053067 discloses that the amount of dihydrotetrabenazine administered may be 10-400 mg per day (although no examples are provided showing that the doses at the lower ends of these ranges are effective) and pharmaceutical compositions containing as little as 10 mg are also described. WO 2006/053067 does not specifically link any particular isomers of dihydrotetrabenazine to these amounts and, more particularly, does not disclose the specific use of 10mg of (+)-α-dihydrotetrabenazine. No experimental data are provided in WO 2006/053067.

WO 2011/153157 (Auspex Pharmaceutical, Inc.) describes deuterated forms of dihydrotetrabenazine. Many deuterated forms of dihydrotetrabenazine are depicted but the application only provides sufficient information to allow a small number of the depicted compounds to be synthesised. Although racemic mixtures of d₆-α-dihydrotetrabenazine and d₆-β-dihydrotetrabenazine are disclosed, these mixtures were not resolved and the properties of the individual (+) and (-) isomers were not studied. Similarly, WO 2014/047167 (Auspex Pharmaceutical, Inc.) describes a number of deuterated forms of tetrabenazine and its derivatives. Again, the individual (+) and (-) isomers of deuterated forms of α- and β-dihydrotetrabenazine were not separated or studied.

It appears therefore that, up to the present, it remains unclear as to precisely which dihydrotetrabenazine isomers are responsible for the therapeutic properties resulting from the administration of tetrabenazine.

It has also remained somewhat unclear up until now whether (+)-α-dihydrotetrabenazine will provide a therapeutically useful effect in the treatment of movement disorders such as Tourette's syndrome without the accompaniment of unwanted side effects such as those described above. Thus, for example, whereas WO2016/127133 (Neurocrine Biosciences) refers to the Kilbourn *et al.* article in Chirality (*idem*) as indicating that (+)-α- dihydrotetrabenazine is the active metabolite of tetrabenazine. WO2016/127133, it also refers to the studies reported in Login et al. (1982), Ann. Neurology 12:257-62 and Reches et al., J. Pharmacol. Exp. Ther. (1983), 225:515-521 which indicate that tetrabenazine inhibits presynaptic and postsynaptic dopamine receptors in the rat brain. It is suggested in WO2016/127133 that this "off-target" activity of tetrabenazine may be responsible for some of the observed side effects of tetrabenazine.

As discussed above, it is known that tetrabenazine exhibits a number of dose-related side effects including causing depression and parkinsonism (see WO2016/127133). It appears that these side-effects may also be caused by VMAT2 inhibition and that consequently it is difficult to separate the therapeutic effect of tetrabenazine and tetrabenazine-derived compounds from these side-effects (see Müller, "Valbenazine granted breakthrough drug status for treating tardive dyskinesia", Expert Opin. Investig. Drugs (2015), 24(6), pp. 737 - 742).

In an attempt to avoid or reduce the side-effects associated with tetrabenazine, a valine ester prodrug of (+)-α-dihydrotetrabenazine has been developed, known by its INN name, Valbenazine. The structure of Valbenazine is shown below:

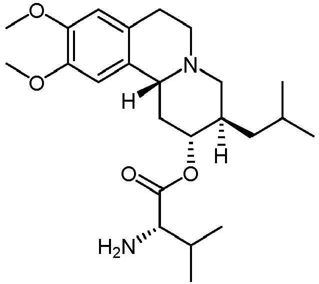

As disclosed in US8039627, Valbenazine is prepared by reacting (+)-α-dihydrotetrabenazine with carbobenzyloxy-L-valine in dichloromethane and 4-dimethylaminopyridine (DMAP) in the presence of N,N'-dicyclohexylcarbodiimide (DCC) to give the intermediate 2-benzyloxycarbonylamino-3-methyl-butyric acid (2R,3R,1bR)-3-isobutyl-9,10-dimethoxy-1,3,4,6,7,11b-hexahydro-2H-pyrido[2,1-a]isoquinolin-2-yl ester. The intermediate is then hydrogenated over palladium on carbon to remove the benzyloxycarbonyl protecting group to give Valbenazine.

Müller ("Valbenazine granted breakthrough drug status for treating tardive dyskinesia", Expert Opin. Investig. Drugs (2015), 24(6), pp. 737 - 742) describes a Phase Ilb clinical study of Valbenazine ("KINECT 1") in patients suffering from tardive dyskinesia. Although some reduction of symptoms was observed when doses of Valbenazine at 100 mg/day (equivalent to about 76 mg of (+)-α-dihydrotetrabenazine) were observed, subjects who received 50 mg/day of Valbenazine (equivalent to about 38 mg of (+)-α-dihydrotetrabenazine) did not show any significant signs of improvement, when scored with the abnormal involuntary movement scale (AIMS). Müller concluded that this study was more or less a failure, probably due to low Valbenazine dosing.

In a further study ("KINECT 2") described in the same paper, subjects were initially dosed at 25 mg/day, with the dose range increasing to 75 mg/day. By the end of the study, when measurements were taken, 21 out of 34 of the subjects treated with Valbenazine were being dosed at 75 mg/day (O'Brien et al, "Kinect 2: NBI-98854 treatment of moderate to severe tardive dyskinesia" Mov. Disord. 2014;29 (Suppl 1):829). The analysis does not provide a breakdown of the reduction in abnormal involuntary movements in patients who were being treated with 75 mg/day by the end of the trial and those who were being treated with 25mg/day or 50mg/day by the end of the trial.

A further Phase III trial of Valbenazine, reported by O'Brien et al ("KINECT 3 A randomised, Double-Blind Placebo-Controlled Phase 3 Trial of Valbenazine (NBI-98854) for Tardive Dyskinesia (PL02.003)", Neurology (2016), 86(16), Supplement PL02.003) investigated the change in abnormal involuntary movements in Tardive Dyskinesia sufferers when administered with 40mg or 80mg of Valbenazine per day. It was found that 80mg/day of Valbenazine resulted in a significant improvement in the Abnormal Involuntary Movement Score and it was concluded that 80mg/day Valbenazine was associated with a significant improvement in Tardive Dyskinesia.

WO 2015/171802 (Neurocrine Biosciences, Inc.) describes methods for treating hyperkinetic diseases by administering therapeutic agents that produce plasma concentrations of (+)-α-dihydrotetrabenazine such that there is a Cₘₐₓ of between about 15ng/ml and 60 ng/ml and a Cₘᵢₙ of at least 15 ng/ml over an eight hour period. Although it is suggested in WO 2015/171802 that this can be accomplished by administering (+)-α-dihydrotetrabenazine *per se,* the experiments described in WO 2015/171802 only provide data for (+)-α-dihydrotetrabenazine levels achieved after the administration of Valbenazine. In Example 1 of WO 2015/171802, it is concluded that a concentration of 30 ng/ml of (+)-α-dihydrotetrabenazine in plasma is an appropriate target and that exposures below 15 ng/ml are suboptimal across the general tardive dyskinesia (TD) population. In Example 2 of WO 2015/171802, it is disclosed that a 50 mg dose of Valbenazine appeared to maintain the required plasma levels of (+)-α-dihydrotetrabenazine.

WO2016/210180 (Neurocrine Biosciences) discloses the use of VMAT2 inhibitors for treating various neurological disorders. (+)-α-dihydrotetrabenazine is mentioned as an example of a VMAT2 inhibitor and therapeutic agents that provide plasma concentrations similar to those disclosed in WO2015/171802 are disclosed. The VMAT2 inhibitory compounds specifically exemplified in WO2016/210180 are Valbenazine and [(2*R*, 3*S*, 11b*R*)-9,10-dimethoxy-3-(2-methylpropyl)-1H,2H,3H,4H,6H,7H,11bH-pyrido[2,1-a]isoquinolin-2-yl]methanol.

Skor et al., "Differences in Dihydrotetrabenazine Isomer Concentrations Following Administration of Tetrabenazine and Valbenazine", Drugs R D, (2017), 17, pp. 449-459 describes the *in vivo* ratios of the diastereoisomers of dihydrotetrabenazine as metabolites of tetrabenazine and Valbenazine following administration of tetrabenazine and Valbenazine. Skor *et al.* conclude that based on the relative abundance and potency of each of the isomers of dihydrotetrabenazine, [+]-β-dihydrotetrabenazine appears to be the primary contributor to VMAT2 inhibition by tetrabenazine. This finding is in contrast with the previously held assertion that [+]-α-dihydrotetrabenazine is the major contributor to the VMAT2 activity of tetrabenazine.

### The Invention

Investigations made by the present applicant indicate that (+)-α-dihydrotetrabenazine *per se* having the chemical name, "(*R*,*R*,*R*)-3-isobutyl-9,10-dimethyloxy-1,3,4,5,7,11b-hexahydro-2H-pyrido[2,1-a]isoquinolin-2-ol", and having the formula (I)

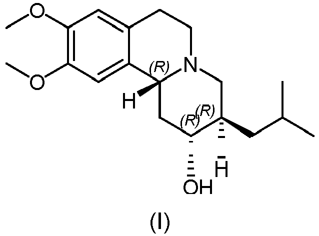

is effective in the treatment of movement disorders at much lower doses than could have been predicted from the literature (for example from WO 2015/171802) and that its use at such lower doses can avoid or minimize the unwanted side effects associated with tetrabenazine.

More particularly, experiments carried out by the present inventors indicate that movement disorders such as Tourette's syndrome can be treated effectively by administering much lower doses of (+)-α-dihydrotetrabenazine *perse* than the doses of Valbenazine required in WO 2015/171802.

In addition, contrary to the teachings of WO 2015/171802, it has been found that by administering (+)-α-dihydrotetrabenazine at a dose to provide blood plasma concentrations that do not exceed 15 ng/mL for a period of 8 hours, efficacious results are achieved.

Furthermore, it has been found that significant VMAT2 binding is observed in humans at times up to 7 hours following administration of low doses of (+)-α-dihydrotetrabenazine. The long-acting effects of these low doses therefore avoid the need for extended-release formulations (see e.g. WO2015/120110) or prodrugs which slowly release (+)-α-dihydrotetrabenazine as their active metabolite (such as Valbenazine - see references above).

This is particularly surprising in view of the recent findings by Skor *et al.* (ibid) which suggest that (+)-β-dihydrotetrabenazine (rather than (+)-α-dihydrotetrabenazine) is the primary contributor to VMAT2 inhibition by tetrabenazine.

The use of lower dosages is expected to reduce the biological side-effects associated with tetrabenazine and tetrabenazine derivatives, including parkinsonism and depression.

An advantage of using (+)-α-dihydrotetrabenazine *per se* rather than the prodrug Valbenazine is that it avoids the two additional synthetic steps and additional purification step required to prepare Valbenazine from (+)-α-dihydrotetrabenazine. In addition, the release of the active (+)-α-dihydrotetrabenazine into the blood plasma is not limited by the rate of degradation of the prodrug.

A further unexpected benefit of using low doses of (+)-α-dihydrotetrabenazine is that experiments conducted by the present inventors suggest that whereas abnormal movements of the type found in movement disorders will be reduced or suppressed by the drug, normal movements will not be. This is in contrast to risperidone, a well-used treatment for movement disorders, where the levels of both normal and abnormal movements can be reduced by administration of the drug.

On the basis of these findings, it is envisaged that low doses of (+)-α-dihydrotetrabenazine will be useful in the prophylaxis or treatment of *inter alia* the disease states and conditions for which tetrabenazine is currently used or proposed. Thus, by way of example, and without limitation, it is envisaged that low doses of (+)-α-dihydrotetrabenazine may be used for the treatment of hyperkinetic movement disorders such as Huntington's disease, hemiballismus, senile chorea, tic disorders, tardive dyskinesia, dystonia and, in particular, Tourette's syndrome.

A1 According to a first embodiment, the present invention relates to a VMAT2 blocking compound for use in a method of treating a subject suffering from a movement disorder; which method comprises administering to the subject a therapeutically effective amount of a VMAT2 blocking compound so as to provide in the subject a therapeutically effective level of VMAT2 blocking; said VMAT2 blocking being maintained such that there is at least 30% VMAT2 blocking after a period of 6.5 hours from administration of the VMAT2 blocking compound; wherein:
- the VMAT2 blocking compound is (+)-α-dihydrotetrabenazine or a pharmaceutically acceptable salt thereof;
- the administration of the therapeutically effective amount of the VMAT2 blocking compound takes place within an initial time window of 30 minutes, and no further administration of the VMAT2 blocking compound takes place within the period of 6.5 hours;
- the said therapeutically effective amount is no more than 8 mg of (+)-α-dihydrotetrabenazine free base or an equimolar amount of a pharmaceutically acceptable salt thereof;
and wherein the VMAT2 blocking compound is presented in an immediate release formulation.

The initial time window is the period within which the (+)-α-dihydrotetrabenazine or a pharmaceutically acceptable salt thereof is administered to the subject. Following the initial time window, no further administration of the VMAT2 blocking compound takes place within a period of 6.5 hours. Whereas, in theory, the (+)-α-dihydrotetrabenazine or salt thereof could be administered in several spaced apart small doses making up the therapeutically effective amount during the initial time window up to 30 minutes, more usually the therapeutically effective amount would be administered over a much shorter period, for example no more than 10 minutes and more typically no more than 5 minutes. Most typically, the (+)-α-dihydrotetrabenazine or a pharmaceutically acceptable salt thereof is administered to the subject during the initial time window in a single dose, and hence the initial time window will typically be no more than about 1 or 2 minutes, although longer initial time windows could be envisaged with particular routes of administration and particular types of dosage form. Furthermore, the use of lower therapeutically effective amounts of the VMAT2 blocking compound can be envisaged.

Accordingly, in further embodiments, the invention provides:
A2 A VMAT2 blocking compound for use according to Embodiment A1 wherein the initial time window is no more than 10 minutes.
A3 A VMAT2 blocking compound for use according to Embodiment A1 wherein the initial time window is no more than 5 minutes.
A4 A VMAT2 blocking compound for use according to Embodiment A1 wherein the initial time window is no more than 2 minutes.
A5. A VMAT2 blocking compound for use according to any one of Embodiments A1 to A4 wherein the VMAT2 blocking compound is administered during the initial time window in a single dose containing the therapeutically effective amount.
A6. A VMAT2 blocking compound for use according to any one of Embodiments A1 to A4 wherein the VMAT2 blocking compound is administered during the initial time window in multiple (e.g. 2 or 3) doses which in aggregate make up the therapeutically effective amount.
A7. A VMAT2 blocking compound for use according to any one of Embodiments A1 to A6 wherein the therapeutically effective amount is no more than 7.5 mg of (+)-α-dihydrotetrabenazine free base or an equimolar amount of a pharmaceutically acceptable salt thereof.
A8. A VMAT2 blocking compound for use according to any one of Embodiments A1 to A6 wherein the therapeutically effective amount is no more than 6 mg of (+)-α-dihydrotetrabenazine free base or an equimolar amount of a pharmaceutically acceptable salt thereof.
A9. A VMAT2 blocking compound for use according to any one of Embodiments A1 to A6 wherein the therapeutically effective amount is no more than 5.5 mg of (+)-α-dihydrotetrabenazine free base or an equimolar amount of a pharmaceutically acceptable salt thereof.
A10. A VMAT2 blocking compound for use according to any one of Embodiments A1 to A6 wherein the therapeutically effective amount is no more than 5 mg of (+)-α-dihydrotetrabenazine free base or an equimolar amount of a pharmaceutically acceptable salt thereof.
A11. A VMAT2 blocking compound for use according to any one of Embodiments A1 to A6 wherein the therapeutically effective amount is in the range from 0.5 mg to 7.5 mg of (+)-α-dihydrotetrabenazine free base or an equimolar amount of a pharmaceutically acceptable salt thereof.
A12. A VMAT2 blocking compound for use according to any one of Embodiments A1 to A6 wherein the therapeutically effective amount is in the range from 1 mg to 7.5 mg of (+)-α-dihydrotetrabenazine free base or an equimolar amount of a pharmaceutically acceptable salt thereof.
A13. A VMAT2 blocking compound for use according to any one of Embodiments A1 to A6 wherein the therapeutically effective amount is in the range from 1 mg to 5 mg of (+)-α-dihydrotetrabenazine free base or an equimolar amount of a pharmaceutically acceptable salt thereof.
A14. A VMAT2 blocking compound for use according to any one of Embodiments A1 to A6 wherein the therapeutically effective amount is in the range from 2 mg to 7.5 mg of (+)-α-dihydrotetrabenazine free base or an equimolar amount of a pharmaceutically acceptable salt thereof.
A15. A VMAT2 blocking compound for use according to any one of Embodiments 1.52-A1 to 1.52-A6 wherein the therapeutically effective amount is in the range from 2 mg to 5 mg of (+)-α-dihydrotetrabenazine free base or an equimolar amount of a pharmaceutically acceptable salt thereof.

The term "immediate release dosage form" is used herein to denote a formulation in which any diluents, carriers or other excipients do not prolong, to an appreciable extent, the rate of drug release and/or absorption: see for example Sandeep et al., Journal of Drug Delivery and Therapeutics; 2013, 3(2), 155-161 and European Pharmacopeia 6.0, part 01/2008: 1502. Such dosage forms are recognised in the art as being distinct from "modified-release", "prolonged-release", "delayed-release" and "pulsatile-release" dosage forms. An immediate release formulation is one which can release a quantity of the active compound (i.e. the (+)-α-dihydrotetrabenazine or a pharmaceutically acceptable salt thereof corresponding to at least 75%, and preferably at least 80% within a defined time, typically 60 minutes or less or more typically 45 minutes or less and most typically 30 minutes or less, as determined according to the test method described in Ph. Eur. 5.17.1 where the paddle speed is set at 50 rpm and an 0.05 M acetate buffer pH 4.5 is used.

The immediate release dosage form can be, for example, a liquid formulation such as a solution (e.g. an aqueous solution of a salt of (+)-α-dihydrotetrabenazine), a syrup, or a suspension (e.g. an aqueous suspension).

Alternatively, the immediate release dosage form can take the form of, for example, a capsule, tablet, lozenge, sublingual wafer (e.g. a melt wafer) or powder.

The immediate release dosage form can be formulated so as to dissolve rapidly in the stomach and thus may be uncoated or bear a coating which dissolves readily in gastric juices.

The immediate release dosage form can be one which disintegrates rapidly in the mouth or when it comes into contact with water. Thus, for example, the immediate release dosage form can take the form of:
(i) a tablet containing a disintegrant (e.g. a superdisintegrant which is a water-swellable cross-linked polymer disintegrant such as cross-linked polyvinylpyrrolidine, sodium starch glycolate or cross-linked carboxymethylcellulose);
(ii) a tablet formed by direct compression (i.e. one which does not contain a granulating agent) and which typically contains a direct compression aid such as microcrystalline cellulose; and optionally a disintegrant (e.g. a superdisintegrant as defined above);
(iii) a chewable dosage form such as a chewable tablet, lozenge or gummie;
(iv) an effervescent tablet containing an effervescent couple; or
(v) a melt.

Dosage forms of type (i) contain a disintegrant and in particular a superdisintegrant which absorbs water and swells rapidly resulting in rapid disintegration of the tablet.

Dosage forms of type (ii) are tablets formed by direct compression of the active compound and any excipients. For many known types of tablet, the active compound is first formed into granules by a wet granulation method involving mixing the compound with a solution of a polymeric granulating (binding) agent such as polyvinylpyrrolidone to bind the particles of active compound together in an agglomerated granule form, which may be milled to provide granules of a defined size range. The granules are then compressed (having optionally first mixed them with other tableting excipients such as diluents and lubricants) to form tablets. However, tablets formed in this way can take an unacceptably long time to disintegrate and release the active compound. In the direct compression dosage tablets of the invention, the wet granulation step and the polymeric binder are omitted and the tablet is formed by compressing a dry formulation containing the active compound and any excipients.

Binding agents and diluents that facilitate tablet formation by direct compression are typically included in the tablets. Such binding agents and diluents include a substance selected from the group consisting of microcrystalline cellulose, silicified microcrystalline cellulose, lactose (e.g. lactose monohydrate), sugar alcohols such as mannitol, erythritol, sorbitol and xylitol, inorganic fillers such as anhydrous calcium salts (e.g. calcium hydrogen phosphate), starches such as maize starch, potato starch, rice starch, wheat starch, pregelatinized starch, fully pregelatinized starch. Particular binding agent and diluents for use in direct compression tablets include pregelatinized starch, microcrystalline cellulose, silicified microcrystalline cellulose, and lactose (e.g. lactose monohydrate). Preferably the direct compression tablets contain microcrystalline cellulose.

The direct compression tablets of the invention typically contain a disintegrant as defined above in (i), and typically also contain a lubricant such as stearic acid, talc, sodium stearyl fumarate or magnesium stearate. Most typically, the lubricant is or comprises magnesium stearate.

The direct compression tablets may contain other excipients as set out below in the "Pharmaceutical Formulations" section of this application provided that such excipients do not have a significant release-retarding effect on the VMAT2 blocking compound.

Unit dosage forms of types (i) and (ii) may each contain a glidant for improving the flow characteristics of the tablet composition prior to compression. Examples of glidants include silicon dioxide, especially colloidal silica, hydrophobic colloidal silica, talc, magnesium silicate and aluminum silicate, particularly fumed silica or Syloid FP silica.

Unit dosage forms of types (i) and (ii) may each comprise:
0.05 to 20 % (w/w), for example up to 5 % (w/w), of (+)-α-dihydrotetrabenazine free base or an equimolar amount of a pharmaceutically acceptable salt thereof;
20 to 90 % (w/w), for example 50 to 90 % (w/w) of a filler, in particular a diluent and/or binding agent disclosed herein;
0.1 to 20 % (w/w), for example 2 to 10 % (w/w) of a disintegrant, in particular a disintegrant as disclosed herein,
0.1 to 5 % (w/w), for example 0.5 to 2 % (w/w) of a lubricant, in particular a lubricant disclosed herein, and
0 to 5 % (w/w), for example up to 2 % (w/w) of a glidant, in particular a glidant disclosed herein.

The immediate release dosage form can be (iii) a chewable dosage form such as a chewable tablet, lozenge or gummie.

Chewable tablets and lozenges, as the names suggest, are tablets and lozenges that are intended to be chewed and broken up in the mouth. As such, they have a lesser hardness than tablets intended to be swallowed and may contain flavouring and/or taste masking agents and optionally substances that improve the mouth feel of the disintegrated chewed tablet. Examples of chewable tablets and the excipients and methods used to make them can be found in, for example, US2014134245 and US5962022. Chewable tablets and lozenges will typically contain a chewable base which may, for example comprise one or more of mannitol, sorbitol, dextrose, fructose or lactose. The tablets or lozenges may also contain conventional lubricants such as magnesium stearate, sweetening agents such as sodium saccharin, aspartame and stevia, and flavouring and colouring agents. The chewable tablets may contain a disintegrant (as defined in (i) above) and/or an effervescent couple (as defined in (iv) above and below) to facilitate disintegration of the tablet.

The immediate release dosage form may be (iv) an effervescent tablet containing an effervescent couple in addition to other conventional excipients such as binders, diluents and lubricants. The effervescent couple comprises a basic ingredient and an acidic ingredient, the basic ingredient liberating carbon dioxide when it and the acidic ingredient are contacted with saliva or added water.

The effervescent couple typically comprises citric acid or sodium hydrogen citrate and sodium bicarbonate, but other physiologically acceptable acid/alkaline or alkaline earth metal carbonate mixtures may be used, for example tartaric, adipic, fumaric or malic acids, and sodium, potassium or calcium (bi)carbonates or sodium glycine carbonate.

The weight of the acidic component may be in the range 0.5% to 20% (w/w), more usually 1.5% to 5% (w/w), of the weight of the tablet. Within these limits, the amount of the effervescent couple is selected at a level sufficient to bring about disintegration of the tablet in the mouth but without itself causing discomfort in the patient's mouth.

The immediate release dosage form can be (iii) a chewable dosage form such as a gummie. Gummies (which may also be referred to as chewable pastilles) are chewable dosage forms that can be chewed without crumbling in the manner that conventional pharmaceutical tablets do when chewed. The properties of the pastilles are thus akin to those of gummi candies. The gummies typically contain, in addition to the active compound, a chewable base comprising a gelling agent and a sugar and/or sugar alcohol and typically also an amount of water. The proportions of the gelling agent and the sugar and the amount of water are selected so that, after processing of the components of the composition, the desired gummy texture is achieved.

The sugar can be a monosaccharide such as glucose or fructose, or a disaccharide such as sucrose, or a mixture of monosaccharide(s) and disaccharide(s).

The sugar alcohol, when present, can be for example selected from glycerol, erythritol, threitol, arabitol, xylitol, ribitol, mannitol, sorbitol, galactitol, fucitol, iditol and inositol, a particular sugar alcohol being xylitol. The sugar alcohol can be used in combination with one or more sugars. For example, in order to reduce the propensity of the compositions to promote tooth decay, at least part of the sugar can be replaced by a sugar alcohol such as xylitol, provided that the ability of the compositions to form a chewable pastille of gummy consistency is not compromised.

In one embodiment, the chewable (gummy) base comprises a gelling agent and a mixture of sucrose and glucose.

The sugar and/or sugar alcohol typically constitute from about 60% (w/w) to about 95% (w/w) of the total weight of the pastille composition.

More usually, the sugar and/or sugar alcohol constitute from about 70% (w/w) to about (90% (w/w) of the composition.

More particularly, the sugar and/or sugar alcohol may constitute from about 75% (w/w) to about 85% (w/w) of the composition.

The gelling agent is a pharmaceutical or food-grade gelling agent and may, for example, be selected from gelatin, pectin, agar, alginate and carrageenan, and mixtures thereof. In one embodiment, the gelling agent is gelatin. In other embodiments, the gelling agent is selected from pectin, agar, alginate or carrageenan, and a mixture of one or more thereof. In one particular embodiment, the gelling agent is pectin.

The gelling agent is typically present in an amount of about 2% (w/w) to about 6% (w/w) of the total weight of the composition. More particularly, the gelling agent may be present in an amount of about 2.5% (w/w) to about 5% (w/w), for example from 3-4% (w/w).

Citric acid may be included in order to assist the gelling agent (particularly pectin) to exert its gelling effect on the sugar. The amount of citric acid may be from 0.1% (w/w) to 5% (w/w), more typically from 0.5% (w/w) to about 3% (w/w), and preferably from 1% to 3% (w/w).

The Immediate release dosage form can also take the form of (v) a melt. Melts ("melt in the mouth" tablets or "MMTs") are solid dosage forms that dissolve or disintegrate rapidly (usually within a minute but often much more quickly) in the oral cavity without the need for water or for chewing. Such tablets are also referred to as orally disintegrating tablets (ODT), fast-dissolving, fast-melting or fast-disintegrating tablets. Guidelines for ODT tablet forms have been produced by the US FDA which defines an ODT to be a solid dosage form containing medicinal substance(s), which disintegrates rapidly, usually within a matter of seconds, when placed upon the tongue.

Melts typically comprise, in addition to the active compound, a diluent or binder (e.g. as defined above), a disintegrant (e.g. as defined in (i) and (ii) above and optionally other pharmaceutical processing aids such as lubricating agents, and are typically formed by direct compression. The disintegration time of the melts can be measured using the procedures set out in the European Pharmacopoeia 6.0, 01/2008:20901. The melts typically disintegrate within a minute.

The immediate release dosage forms can also be provided in liquid form, for example as solutions, syrups or suspensions for oral administration.

Solution formulations contain a pharmaceutically acceptable solvent which may be, for example, an aqueous or alcoholic solvent and may comprise one or more solvents selected from water, ethanol, glycerine and propylene glycol and mixtures thereof. The solutions may be aqueous solutions and will usually contain a watersoluble salt form of (+)-α-dihydrotetrabenazine, for example a salt form as defined herein. The solution formulations may contain one or more excipients selected from sugars, sugar alcohols, preservatives, flavouring agents, anti-oxidants and pharmaceutical grade surfactants, the nature of each of which will be readily known to the skilled person.

Syrup formulations typically contain a syrup base formed from a sugar such as glucose or sucrose and a flavouring agent (e.g. a fruit flavouring) as well as one or more other standard pharmaceutical excipients as defined herein.

Suspension formulations typically contain (+)-α-dihydrotetrabenazine or a pharmaceutically acceptable salt thereof in a finely divided particulate state (e.g. wherein the particles have a D₉₀ of less than 100 µm) suspended in a liquid medium which is typically an aqueous medium. The suspension formulation will typically contain one or more suspending agents which serve to stabilize the suspension and prevent the particulate active compound from separating out.

Suspension formulations can be either buffered or unbuffered.

Examples of suspending agents include xanthan gum, hydroxypropylmethylcellulose, methylcellulose, carageenan, sodium carboxymethyl cellulose, and sodium carboxymethyl cellulose/ microcrystalline cellulose mixtures, particularly sodium carboxymethyl cellulose/ microcrystalline cellulose mixtures. Particular suspending agents are thixotropic suspending agents such as xanthan gum. carageenan and sodium carboxymethyl cellulose/microcrystalline cellulose mixtures. The amount of suspending agent present will vary according to the particular suspending agent used and the presence or absence of other ingredients which have an ability to act as a suspending agent or which contribute significantly to the. viscosity of the composition. In general, however, the amount of suspending agent will lie in the range 0.1-1.5% w/w of the total weight of the composition. When the suspending agent is xanthan gum, it will usually be present in an amount corresponding to 0.1-0.5% w/w of the total weight whereas when a sodium carboxymethyl cellulose/microcrystalline cellulose mixture is used, the amount typically will lie in the range 0.6-1.5% w/w, particularly approximately 1.2% w/w. When carageenan is used, typically this will constitute 0.5-1 % w/w of the composition. Compositions containing alginate, which has a significant thickening effect, will, in general, contain lower concentrations of suspending agent in order to avoid the problem of the viscosity being so great that the composition cannot be poured.

The suspension can contain ingredients which improve its taste, for example sweeteners, bitter-taste maskers such as sodium chloride and taste-masking flavours such as contramarum, flavour enhancers such as monosodium glutamate, and flavouring agents.

Examples of sweeteners include bulk sweeteners such as sucrose, hydrogenated glucose syrup, the sugar alcohols sorbitol and xylitol. and sweetening agents such as sodium cyclamate, sodium saccharin, aspartame and ammonium glycyrrhizinate.

A bulk sweetener will usually be present in an amount corresponding to about 15-70% w/w of the total weight of the suspension, the amount depending in part upon whether other ingredients, e.g. alginate, are present which have a thickening effect on the composition. For example, when sorbitol is used as the sole bulk sweetener and no thickener (e.g. alginate) is present, typically the dry weight of sorbitol present is in the range 35-55% w/w of the total weight of the suspension, for example at a concentration of approximately 45% w/w.

When hydrogenated glucose syrup (solids content approximately 74%) is used as the sole bulk sweetener, typically it is present as 55-70% w/w of the suspension, for example at a concentration of approximately 65% w/w (equivalent to 49% solids). It will be appreciated that combinations of bulk sweeteners can be used, for example combinations of sorbitol and hydrogenated glucose syrup, or sucrose and sorbitol.

Other excipients which can be used include humectants such as propylene glycol and glycerol and pigments such as titanium dioxide.

Typically the total quantity of humectant present is in the range 0-10% w/w. Thus, for example, propylene glycol and glycerol can each be present in an amount approximating to 4% w/w. It is preferred that the suspensions contain preservatives to prevent microbial contamination. Examples of preservatives are the alkylparabens, particularly propylparaben and butylparaben.

The immediate release dosage form may also be in the form of a capsule. The capsules may be hard-shelled capsules or soft-shelled capsules. The capsules may be made from gelling agents, such as animal protein (e.g. gelatin) or plant-based alternatives including plant polysaccharides. Hard-shelled capsules may be of conventional type and may comprise a capsule body and a cap, the cap being fitted onto the capsule body after filling.

Hard-shelled (e.g. hard gelatine) capsules may comprise the (+)-α-dihydrotetrabenazine or a pharmaceutically acceptable salt thereof with a diluent (such as lactose) in a non-compressed (e.g. powdered) form with diluent. The capsule may also comprise a lubricant (such as magnesium stearate). Such capsules dissolve readily in gastric juices thereby providing rapid release of the (+)-α-dihydrotetrabenazine or salt thereof.

Hard shelled (e.g. hard gelatin) capsules can also be used to deliver semi-solid compositions comprising (+)-α-dihydrotetrabenazine or a pharmaceutically acceptable salt thereof dispersed or dissolved in a hydrophilic carrier, such as a polyethylene glycol. Such compositions can be prepared by melting a carrier such as polyethylene glycol, dissolving or dispersing the active compound in the molten carrier, filling (e.g. pumping) the resulting mixture into a capsule body so that the mixture cools to form a semi-solid, and sealing the capsule body by, for example, means of a cap. As the carrier is hydrophilic, the carrier will dissolve rapidly upon contact with water and rapidly release the (+)-α-dihydrotetrabenazine or salt thereof.

Hard gelatin capsules can also be used to deliver solutions and suspensions. In this case, the (+)-α-dihydrotetrabenazine is dispersed in a diluent and pumped into the capsule. The capsule may then be hermetically sealed or be provided with a ring of gelatin subsequently applied to an area of the hard gelatin capsule where cap and body coincide.

For soft gelatin capsules, the (+)-α-dihydrotetrabenazine or a pharmaceutically acceptable salt thereof may be dissolved or dispersed in a non-aqueous diluent. The diluent and (+)-α-dihydrotetrabenazine are then filled into a soft gelatin capsule, which is subsequently sealed.

Equipment for filling and sealing hard-shelled and soft-shelled capsules is well known to those skilled in the art.

Capsules may contain a filling in the form of a powder, immediate release pellets, immediate release microbeads or immediate release mini-tablets containing the (+)-α-dihydrotetrabenazine or a pharmaceutically acceptable salt thereof along with diluents (e.g. sugars and sugar alcohols described herein) and other excipients as described herein provided that such excipients do not retard release of the VMAT2 blocking compound. Capsules may alternatively contain a filling in the form of powdered pure (+)-α-dihydrotetrabenazine or a pharmaceutically acceptable salt but this is less preferred and more usually one or more diluents or other excipients will be present. Typically, the capsules are not provided with an acid-resistant or enteric coating. The capsules may be provided in a blister pack or other suitable container.

Alternatively, the immediate release dosage form may be in the form of a powder, the powder comprising (+)-α-dihydrotetrabenazine or a pharmaceutically acceptable salt thereof and one or more diluents and/or other excipients as described herein (for example a sugar or sugar alcohol as described herein). The powder may optionally comprise a flavouring agent. The powder may be presented in a container (such as a sachet) which can be opened (e.g. torn - in the case of a sachet) to allow access to the powder.

The immediate release dosage form may also be in the form of a liquid formulation for intranasal administration. The liquid formulation may be in the form of a solution or a colloidal suspension (and more usually a solution). The solution or suspension comprises (+)-α-dihydrotetrabenazine or a pharmaceutically acceptable salt thereof and an appropriate liquid diluent such as water, optionally together with one or more other excipients such as tonicity adjusters (e.g. sodium chloride), pH adjusting agents, preservatives, buffering agents, surfactants, suspending agents and co-solvents. The solution/suspension can be presented in in a container which is provided with a metered spray pump or metered aerosol delivery device.

In each of the immediate release dosage forms defined above, where the (+)-α-dihydrotetrabenazine or a pharmaceutically acceptable salt thereof is in a solid (i.e. non-dissolved) form, the (+)-α-dihydrotetrabenazine or its salt may be in a finely divided particulate form in order to facilitate rapid dissolution and absorption. Thus, for example, the (+)-α-dihydrotetrabenazine or a pharmaceutically acceptable salt may be present in the form of particles having a D₉₀ value of less than 250 µm, more usually less than 250 µm, for example in the range from 40 to 150 µm as determined by laser diffraction (for example using a Malvern Mastersizer laser diffraction instrument).

In further embodiments of the invention, the VMAT2 blocking is maintained such that there is at least 30% VMAT2 blocking after a period of more than 6.5 hours (for example a period of 7 hours, 7.5 hours, 8 hours, 8.5 hours, 9 hours, 9.5 hours or 10 hours) from administration of the VMAT2 blocking compound, and no further administration of the VMAT2 blocking compound takes place within the said period.

Accordingly, in further embodiments there is provided:
A16. A VMAT2 blocking compound for use according to any one of Embodiments A1 to A15 wherein the (+)-α-dihydrotetrabenazine is administered in the form of a tablet comprising (+)-α-dihydrotetrabenazine or a pharmaceutically acceptable salt thereof and a disintegrant.
A17. A VMAT2 blocking compound for use according to any one of Embodiments A1 toA15 wherein the (+)-α-dihydrotetrabenazine or a pharmaceutically acceptable salt thereof is administered in the form of a tablet formed by direct compression.
A18. A VMAT2 blocking compound for use according to any one of Embodiments A1 to A15 wherein the (+)-α-dihydrotetrabenazine or a pharmaceutically acceptable salt thereof is administered in the form of a chewable dosage form such as a chewable tablet, lozenge or gummie.
A19. A VMAT2 blocking compound for use according to any one of Embodiments A1 to A15 wherein the (+)-α-dihydrotetrabenazine or a pharmaceutically acceptable salt thereof is administered in the form of an effervescent tablet containing an effervescent couple.
A20. A VMAT2 blocking compound for use according to any one of Embodiments A1 to A15 wherein the (+)-α-dihydrotetrabenazine or a pharmaceutically acceptable salt thereof is administered in the form of a melt.
A21. A VMAT2 blocking compound for use according to any one of Embodiments A1 to A15 wherein the (+)-α-dihydrotetrabenazine or a pharmaceutically acceptable salt thereof is administered in the form of a solution.
A22. A VMAT2 blocking compound for use according to any one of Embodiments A1 to A15 wherein the (+)-α-dihydrotetrabenazine or a pharmaceutically acceptable salt thereof is administered in the form of a syrup.
A23. A VMAT2 blocking compound for use according to any one of Embodiments A1 to A15 wherein the (+)-α-dihydrotetrabenazine or a pharmaceutically acceptable salt thereof is administered in the form of a suspension.
A24. A VMAT2 blocking compound for use according to any one of Embodiments A1 to A15 wherein the (+)-α-dihydrotetrabenazine or a pharmaceutically acceptable salt thereof is administered in the form of a sublingual tablet, wafer or spray.
A25. A VMAT2 blocking compound for use according to any one of Embodiments A1 to A15 wherein the (+)-α-dihydrotetrabenazine or a pharmaceutically acceptable salt thereof is administered in the form of a capsule.
A26. A VMAT2 blocking compound for use according to Embodiment A25 wherein the capsule is a hard-shelled (e.g. hard gelatin) capsule.
A27. A VMAT2 blocking compound for use according to Embodiment A25 wherein the capsule is a soft-shelled (e.g. soft gelatin) capsule.
A28. A VMAT2 blocking compound for use according to any one of Embodiments A1 to A15 wherein the (+)-α-dihydrotetrabenazine or a pharmaceutically acceptable salt thereof is administered in the form of a powder.
A29. A VMAT2 blocking compound for use according to any one of Embodiments A1 to A15 wherein the (+)-α-dihydrotetrabenazine or a pharmaceutically acceptable salt thereof is administered in the form of an intranasal spray.
A30 A VMAT2 blocking compound for use according to any one of Embodiments A1 to A29 wherein the (+)-α-dihydrotetrabenazine or a pharmaceutically acceptable salt thereof is in the form of particles having a D₉₀ value of less than 250 µm, more usually less than 250 µm, for example in the range from 40 to 150 µm as determined by laser diffraction (for example using a Malvern Mastersizer laser diffraction instrument).

In each of Embodiments A1 to A30, the (+)-α-dihydrotetrabenazine or pharmaceutically acceptable salt thereof is not administered in combination with a therapeutically effective amount of amantadine, and more preferably is not administered in combination with any amount of amantadine.

A VMAT2 blocking compound for use defined and described above are typically for use in the treatment of a hyperkinetic movement disorder such as Huntington's disease, hemiballismus, senile chorea, tic disorders, tardive dyskinesia, dystonia, myoclonus and Tourette's syndrome.

More particularly, the unit dosage forms described above are for use in the treatment of a hyperkinetic movement disorder selected from tic disorders, tardive dyskinesia and Tourette's syndrome.

In one particular embodiment, the unit dosage forms described above are for use in the treatment of tardive dyskinesia.

In another particular embodiment, the unit dosage forms described above are for use in the treatment of Tourette's syndrome.

The term "treatment" as used herein in the context of treating a condition or disorder, pertains generally to treatment and therapy in which some desired therapeutic effect is achieved, for example, the inhibition of the progress of the condition, and includes a reduction in the rate of progress, a halt in the rate of progress, amelioration of the condition, diminishment or alleviation of at least one symptom associated or caused by the condition being treated and cure of the condition. When the hyperkinetic movement disorder being treated is Tourette's Syndrome, treatment of the disorder may pertain to a reduction of the incidence or severity of tics.

Accordingly, in further embodiments (Embodiments 1.85 to 1.88), the invention also provides:
1.85 A VMAT2 blocking compound for use as described herein wherein the movement disorder is selected from tardive dyskinesia, Tourette's syndrome and Huntington's disease.
1.86 A VMAT2 blocking compound for use according to Embodiment 1.85 wherein the movement disorder is Tourette's Syndrome.
1.87 A VMAT2 blocking compound for use according to Embodiment 1.85 wherein the movement disorder is Huntington's Disease.
1.88 A VMAT2 blocking compound for use according to Embodiment 1.85 wherein the movement disorder is tardive dyskinesia.

In each case, unless the context indicates otherwise, the quantity of (+)-α-dihydrotetrabenazine specified may be administered once per day or in several (e.g. two) doses per day.

In some embodiments, the quantity of (+)-α-dihydrotetrabenazine specified is administered once daily.

The administration of (+)-α-dihydrotetrabenazine typically forms part of a chronic treatment regime. The (+)-α-dihydrotetrabenazine may therefore be administered to a patient for a treatment period of at least a week, more usually at least two weeks, or at least a month, and typically longer than a month. Where a patient is shown to respond well to treatment, the period of treatment can be longer than six months and may extend over a period of years.

The chronic treatment regime may involve the administration of the (+)-α-dihydrotetrabenazine every day, or the treatment regime may include days when no (+)-α-dihydrotetrabenazine is administered.

The dosage administered to the subject may vary during the treatment period. For example, the initial dosage may be increased or decreased depending on the subject's response to the treatment. A subject may, for example, be given an initial low dose to test the subject's tolerance towards the (+)-α-dihydrotetrabenazine, and the dosage thereafter increased as necessary up to the maximum daily intake of 20 mg. Alternatively, an initial daily dosage administered to the patient may be selected so as to give an estimated desired degree of VMAT2 blockage, following which a lower maintenance dose may be given for the remainder of the treatment period, with the option of increasing the dosage should the subject's response to the treatment indicate that an increase is necessary.

In each of the foregoing aspects and embodiments, the (+)-α-dihydrotetrabenazine can be administered as the free base or as a pharmaceutically acceptable salt. In one embodiment, the (+)-α-dihydrotetrabenazine is administered as a pharmaceutically acceptable salt. In another embodiment, the (+)-α-dihydrotetrabenazine is administered as a free base. The quantities of (+)-α-dihydrotetrabenazine are calculated as the amounts of the free base, or when the (+)-α-dihydrotetrabenazine is in the form of a pharmaceutically acceptable salt, the amount of (+)-α-dihydrotetrabenazine free base present in the pharmaceutically acceptable salt.

The present inventors have found that plasma levels of (+)-α-dihydrotetrabenazine required for effective treatment of hyperkinetic movement disorders can be considerably lower than the plasma levels achieved by administration of Valbenazine as described in WO 2015/171802.

Accordingly, in a further embodiment, the invention provides:
- (+)-α-dihydrotetrabenazine, or a pharmaceutically acceptable salt thereof, for use in a method of treatment of a movement disorder wherein the treatment comprises administering to a subject a therapeutically effective amount of the (+)-α-dihydrotetrabenazine or pharmaceutically acceptable salt thereof, in an amount sufficient to achieve an average blood plasma C_{avg} concentration, where measured over the period of five hours in the range from 2 ng/ml to 15 ng/ml.

In one embodiment, the invention provides:
- (+)-α-dihydrotetrabenazine, or a pharmaceutically acceptable salt thereof, for use in a method of treatment of a movement wherein the treatment comprises administering to a subject a therapeutically effective amount of the (+)-α-dihydrotetrabenazine or pharmaceutically acceptable salt thereof, in an amount sufficient to achieve an average blood plasma C_{avg} concentration, when measured over a period of three hours, in the range from 3 ng/ml to 15 ng/ml.

Complete blocking of VMAT2 is considered undesirable as this can lead to unwanted side effects, such as Parkinsonism. The present invention provides plasma levels of (+)-α-dihydrotetrabenazine that are sufficient to give effective treatment of movement disorders but do not block VMAT2 to an extent that causes Parkinsonism and similar side effects. The levels of VMAT2 blocking can be determined by competitive binding studies using Positron Emission Tomography (PET). By co-administering a radioactive ligand with the compound of interest at various concentrations, the proportion of binding sites occupied can be determined (see for example, Matthews et al., "Positron emission tomography molecular imaging for drug development", Br. J. Clin. Pharmacol., 73:2, 175-186).

Accordingly, in further embodiments (Embodiments 1.125 to 1.147), there is provided:
1.125 A VMAT2 blocking compound for use as described herein, wherein the treatment comprises administering to the subject an amount of (+)-α-dihydrotetrabenazine sufficient to cause a level of blocking of up to 85% of the VMAT2 proteins in the subject.
1.126 A VMAT2 blocking compound for use as described herein, wherein the treatment comprises administering to the subject an amount of (+)-α-dihydrotetrabenazine sufficient to cause a level of blocking of up to 80% of the VMAT2 proteins in the subject.
1.127 A VMAT2 blocking compound for use as described herein, wherein the treatment comprises administering to the subject an amount of (+)-α-dihydrotetrabenazine sufficient to cause a level of blocking of up to 75% of the VMAT2 proteins in the subject.
1.128 A VMAT2 blocking compound for use as described herein, wherein the treatment comprises administering to the subject an amount of (+)-α-dihydrotetrabenazine sufficient to cause a level of blocking of up to 70% of the VMAT2 proteins in the subject.
1.129 A VMAT2 blocking compound for use as described herein, wherein the treatment comprises administering to the subject an amount of (+)-α-dihydrotetrabenazine sufficient to cause a level of blocking of from 25% to 85% of the VMAT2 proteins in the subject.
1.130 A VMAT2 blocking compound for use as described herein, wherein the treatment comprises administering to the subject an amount of (+)-α-dihydrotetrabenazine sufficient to cause a level of blocking of from 30% to 80% of the VMAT2 proteins in the subject.
1.131 A VMAT2 blocking compound for use as described herein, wherein the treatment comprises administering to the subject an amount of (+)-α-dihydrotetrabenazine sufficient to cause a level of blocking of from 35% to 75% of the VMAT2 proteins in the subject.
1.132 A VMAT2 blocking compound for use as described herein, wherein the treatment comprises administering to the subject an amount of (+)-α-dihydrotetrabenazine sufficient to cause a level of blocking of from 35% to 70% of the VMAT2 proteins in the subject.
1.133 A VMAT2 blocking compound for use as described herein, wherein the treatment comprises administering to the subject an amount of (+)-α-dihydrotetrabenazine sufficient to cause a level of blocking of from 40% to 75% of the VMAT2 proteins in the subject.
1.134 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of from 45% to 75% of the VMAT2 proteins in the subject.
1.135 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of from 35% to 80% of the VMAT2 proteins in the subject.
1.136 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of from 40% to 80% of the VMAT2 proteins in the subject.
1.137 A VMAT2 blocking compound for use as described herein, wherein the treatment comprises administering to the subject an amount of (+)-α-dihydrotetrabenazine sufficient to cause a level of blocking of from 45% to 80% of the VMAT2 proteins in the subject.
1.138 A VMAT2 blocking compound for use as described herein, wherein the treatment comprises administering to the subject an amount of (+)-α-dihydrotetrabenazine sufficient to cause a level of blocking of from 50% to 80% of the VMAT2 proteins in the subject.
1.139 A VMAT2 blocking compound for use as described herein, wherein the treatment comprises administering to the subject an amount of (+)-α-dihydrotetrabenazine sufficient to cause a level of blocking of from 55% to 80% of the VMAT2 proteins in the subject.
1.140 A VMAT2 blocking compound for use as described herein, wherein the treatment comprises administering to the subject an amount of (+)-α-dihydrotetrabenazine sufficient to cause a level of blocking of VMAT2 proteins in the subject of between 30% and 70%.
1.141 A VMAT2 blocking compound for use as described herein, wherein the treatment comprises administering to the subject in need thereof, wherein the method comprising administering to a subject an amount of (+)-α-dihydrotetrabenazine sufficient to cause a blocking level of VMAT2 proteins in the subject of between 30% and 65%.
1.142 A VMAT2 blocking compound for use as described herein, wherein the treatment comprises administering to the subject in need thereof, wherein the method comprising administering to a subject an amount of (+)-α-dihydrotetrabenazine sufficient to cause a blocking level of VMAT2 proteins in the subject of between 30% and 60%.
1.143 A VMAT2 blocking compound for use as described herein, wherein the treatment comprises administering to the subject in need thereof, wherein the method comprising administering to a subject an amount of (+)-α-dihydrotetrabenazine sufficient to cause a level blocking of VMAT2 proteins in the subject of between 40% and 80%.
1.144 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of VMAT2 proteins in the subject of between 40% and 75%.
1.145 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of VMAT2 proteins in the subject of between 40% and 70%.
1.146 A VMAT2 blocking compound for use as described herein, wherein the treatment comprises administering to the subject in need thereof, wherein the method comprising administering to a subject an amount of (+)-α-dihydrotetrabenazine sufficient to cause a level of blocking of VMAT2 proteins in the subject of between 40% and 65%.
1.147 A VMAT2 blocking compound for use as described herein, wherein the treatment comprises administering to the subject in need thereof, wherein the method comprising administering to a subject an amount of (+)-α-dihydrotetrabenazine sufficient to cause a level blocking of VMAT2 proteins in the subject of between 40% and 60%.

In the methods and uses described herein, the amounts of (+)-α-dihydrotetrabenazine or a pharmaceutically acceptable salt required to be administered to a subject may depend in part on the speed of metabolism of the compound by the subject.

Metabolism of many drugs (approximately 25% of all current prescription drugs) involves the cytochrome P450 2D6 (CYP2D6) enzyme which is primarily expressed in the liver.

Approximately 7% of the population has a slow acting form of this enzyme and 7% a super-fast acting form. Thirty-five percent are carriers of a non-functional CYP2D6 allele.

As a result, subjects can be classified according to whether they are:
- poor metabolizers - with little or no CYP2D6 function;
- intermediate metabolizers - who metabolize drugs at a rate somewhere between the poor and extensive metabolizers;
- extensive metabolizers - with normal CYP2D6 function; or
- ultrarapid metabolizer - with multiple copies of the CYP2D6 gene are expressed, so greater-than-normal CYP2D6 function occurs.

Thus, considerable variation exists in the efficiency and amount of CYP2D6 enzyme produced between individuals. Hence, for drugs that are metabolized by CYP2D6, certain individuals will eliminate these drugs quickly (ultrarapid metabolizers) while others will do so slowly (poor metabolizers). If a drug is metabolized too quickly, it may decrease the drug's efficacy while if the drug is metabolized too slowly, toxicity may result.

A number of commercially available diagnostic tests are available for determining whether a subject is a fast or slow metabolizer and these include the CYP2D6 Genotyping tests available from Genelex Labs LLC, Seattle, WA 98121, USA, and Trimgen Corporation, Sparks, Maryland 21152, USA.

Therefore, in each of the methods and uses described herein the use or method may include the step of testing a subject to determine the CYP2D6 status of the subject and then using the outcome of the test as a factor in determining the amounts of (+)-α-dihydrotetrabenazine or a pharmaceutically acceptable salt to be administered to the subject. Thus, a fast metabolizer may be administered an amount towards the upper end of a given range whereas a slower metabolizer may be administered a smaller amount.

In each of the foregoing embodiments, the unit dosage form (or the substance administered in the method) contains no more than 20% by weight, relative to the (+)-α-dihydrotetrabenazine, of any other isomer of dihydrotetrabenazine; more usually contains no more than 10% by weight, relative to the (+)-α-dihydrotetrabenazine, of any other isomer of dihydrotetrabenazine; preferably contains no more than 5% by weight, relative to the (+)-α-dihydrotetrabenazine, of any other isomer of dihydrotetrabenazine; and more preferably contains no more than 2% by weight, relative to the (+)-α-dihydrotetrabenazine, of any other isomer of dihydrotetrabenazine.

Thus, the (+)-α-dihydrotetrabenazine typically has an isomeric purity of at least 80%.

The term "isomeric purity" in the present context refers to the amount (+)-α-dihydrotetrabenazine present relative to the total amount or concentration of dihydrotetrabenazines of all isomeric forms. For example, if 90% of the total dihydrotetrabenazine present in the composition is (+)-α-dihydrotetrabenazine then the isomeric purity is 90%.

The (+)-α-dihydrotetrabenazine of the invention may have an isomeric purity of greater than 82%, greater than 85%, greater than 87%, greater than 90%, greater than 91%, greater than 92%, greater than 93%, greater than 94%, greater than 95%, greater than 96%, greater than 97%, greater than 98%, greater than 99%, greater than 99.5%, or greater than 99.9%.

### Salts

The dihydrotetrabenazine may be presented as the free base or in the form of salts. All references herein to (+)-α-dihydrotetrabenazine include (+)-α-dihydrotetrabenazine in both free base and salt forms thereof, unless the context indicates otherwise.

In one embodiment, the (+)-α-dihydrotetrabenazine as defined herein is in the form of the free base.

In another embodiment, the (+)-α-dihydrotetrabenazine as defined herein is in the form of a salt.

The salts are typically acid addition salts.

The salts can be synthesized from the parent compound by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free base form of the compound with the acid in water or in an organic solvent, or in a mixture of the two; generally, nonaqueous media such as ether, ethyl acetate, ethanol, isopropanol, or acetonitrile are used.

Acid addition salts may be formed with a wide variety of acids, both inorganic and organic. Examples of acid addition salts include salts formed with an acid selected from the group consisting of benzenesulphonic, camphor-sulphonic, (+)-(1S)-camphor-10-sulphonic, di-p-toluoyl-D-tartaric acid, ethane-1,2-disulphonic, ethanesulphonic, 2-hydroxyethanesulphonic, hydrochloric, naphthalene-2-sulphonic, naphthalene-1,5-disulphonic, nitric, phosphoric, succinic, sulphuric, and *p*-toluenesulphonic acids. Due to its enhanced solubility in aqueous solvents (see International Patent Application No. PCT/EP2018/058109), in a preferred embodiment, the (+)-α-dihydrotetrabenazine for use as described herein is in the form of the succinate salt.

The salt forms of the compound of the invention are typically pharmaceutically acceptable salts, and examples of pharmaceutically acceptable salts are discussed in Berge et al., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention.

### Isotopes

The (+)-α-dihydrotetrabenazine may contain one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹¹C, ¹²C, ¹³C and ¹⁴C and ¹⁶O and ¹⁸O.

Typically, the (+)-α-dihydrotetrabenazine of the invention does not contain isotopes (such as ¹¹C or ³H) in amounts higher than their natural abundance.

In one embodiment, the percentage of the total hydrogen atoms in the (+)-α-dihydrotetrabenazine that are deuterium atoms is less than 2%, more typically less than 1%, more usually less than 0.1%, preferably less than 0.05% and most preferably no more than 0.02%.

In an analogous manner, a reference to a particular functional group also includes within its scope isotopic variations, unless the context indicates otherwise.

The isotopes may be radioactive or non-radioactive. In one embodiment of the invention, the (+)-α-dihydrotetrabenazine contains no radioactive isotopes. Such compounds are preferred for therapeutic use. In another embodiment, however, the (+)-α-dihydrotetrabenazine may contain one or more radioisotopes. Compounds containing such radioisotopes may be useful in a diagnostic context.

### Solvates

(+)-α-Dihydrotetrabenazine may form solvates.

Examples of solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulphoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGE), differential scanning calorimetry (DSC) and X-ray crystallography.

The solvates can be stoichiometric or non-stoichiometric solvates.

Particular solvates are hydrates, and particular examples of hydrates include hemihydrates, monohydrates and dihydrates.

For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.

Alternatively, rather than existing as a hydrate, the compound of the invention may be anhydrous. Therefore, in another embodiment, the (+)-α-dihydrotetrabenazine is in an anhydrous form.

### Methods for the Preparation of (+)-α-Dihydrotetrabenazine

(+)-α-Dihydrotetrabenazine (compound of formula (I)) can be prepared from tetrabenazine according to the synthetic route shown in Scheme 1.

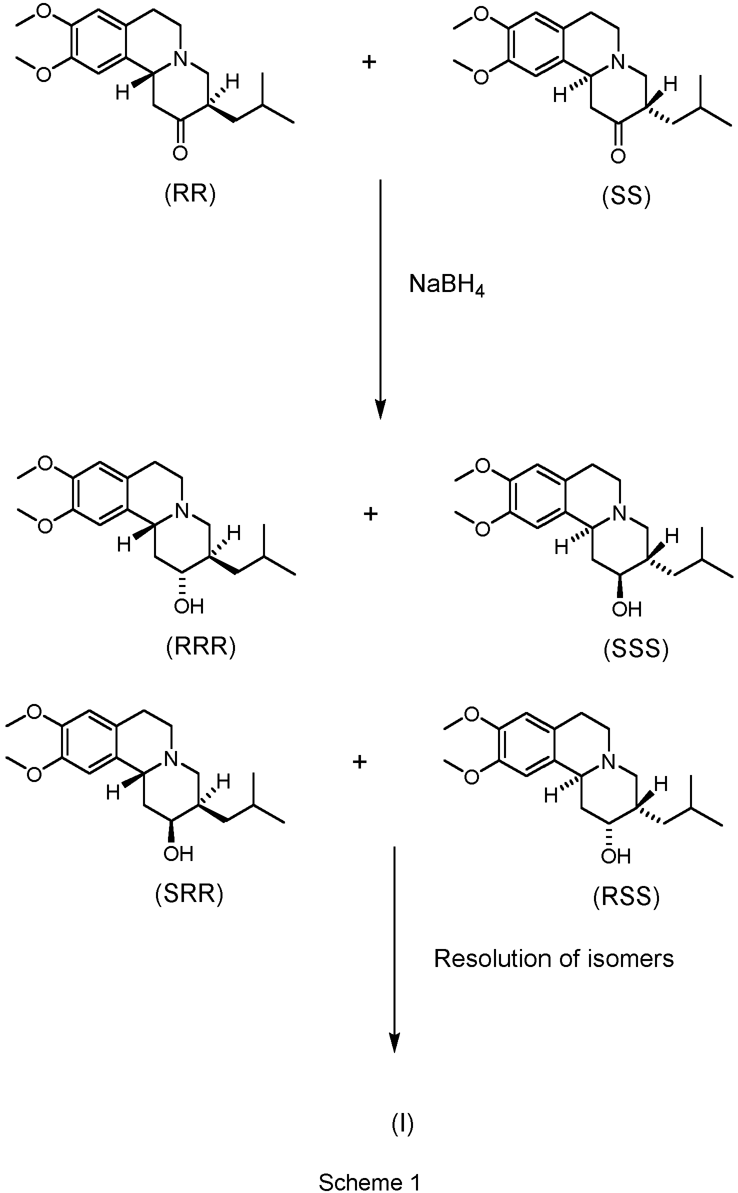

Racemic tetrabenazine (3-isobutyl-9,10-dimethoxy-1,3,4,6,7,11 b-hexahydro-2*H-*pyrido[2,1,a]isoquinolin-2-one) containing the *RR* and *SS* isomers of tetrabenazine is reduced with sodium borohydride to afford a mixture of four dihydrotetrabenazine isomers of which a racemic mixture of the α-dihydrotetrabenazines (*RRR* and SSS isomers) constitutes the major product and a racemic mixture of the β-dihydrotetrabenazines (the *SRR* and *RSS* isomers) constitutes a minor product. The β-dihydrotetrabenazines can be removed during an initial purification procedure, for example by chromatography or recrystallization and then the racemic α-dihydrotetrabenazines resolved (e.g. by recrystallisation with di-p-toluoyl-L-tartaric acid or (R)-(-)-camphorsulfonic acid or by chiral chromatography), to afford (+)-α-dihydrotetrabenazine (I) ((2*R*, *3R, 11bR)*-3-isobutul-9,10-dimethox-1,3,4,6,7,11 b-hexahydro-2*H*-pyrido[2,1,a]isoquinolin-2-ol). The stereochemical configuration of (+)-α-dihydrotetrabenazine can be determined, for example by forming the compound or a salt thereof in crystalline form and identifying the structure by X-ray crystallography.

(+)-α-Dihydrotetrabenazine can also be prepared according to Yao et al., "Preparation and evaluation of tetrabenazine enantiomers and all eight stereoisomers of dihydrotetrabenazine as VMAT2 inhibitors", Eur. J. Med. Chem., (2011), 46, pp. 1841 - 1848.

### Pharmaceutical Formulations

In each of embodiments described herein, where the compound is administered as a pharmaceutical unit dosage form, or other pharmaceutical composition, the pharmaceutical compositions of the invention can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules, caplets, pills, lozenges, syrups, solutions, sprays, powders, granules, elixirs and suspensions, sublingual tablets, sprays, wafers or patches and buccal patches. In one embodiment, the dosage form is a tablet. In another embodiment, the dosage form is a capsule.

A particular group of pharmaceutical dosage forms for use as described herein consists of tablets, capsules, solutions, syrups, suspensions and gels.

Pharmaceutical compositions containing the dihydrotetrabenazine compound of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

In each of the foregoing embodiments, the solid dosage forms (e.g. capsules or tablets) can be provided in suitable packaging for supplying the dosage forms to a patient. Such packaging includes bottles (e.g. child-resistant medicine bottle) and blister packs.

Blister packs typically comprise a plurality of cavities or pockets in a thermoformed plastic with a lidding seal of aluminum foil or plastic film. Medicine bottles may be formed from glass or plastic and comprise a container, a removable lid and optionally a foil seal (such as a tamperproof) for sealing the opening in the container.

In view of the studies carried out by the inventors, it is envisaged that the administration of low doses of (+)-α-dihydrotetrabenazine are sufficient for establishing therapeutically effective levels of VMAT2 blocking for periods in excess of 7 hours (e.g. up to 12 hours). Accordingly, the (+)-α-dihydrotetrabenazine for use in the invention may be delivered in an immediate release dosage form.

For example, the (+)-α-dihydrotetrabenazine may be administered in a pharmaceutical formulation (e.g. a tablet) comprising (+)-α-dihydrotetrabenazine and a pharmaceutically acceptable diluent or carrier, wherein the diluent or carrier does not prolong, (at least to an appreciable extent), the rate of release and/or absorption of (+)-α-dihydrotetrabenazine. Examples of suitable diluents/carriers include disintegrants such as cross-linked polyvinylpyrrolidone, crospovidone, sodium starch glycolate and carboxymethylcellulose. These disintegrants provide rapid disintegration of the tablet in the stomach following oral administration.

The (+)-α-dihydrotetrabenazine may be administered in a capsule with an acid soluble coating in order to delay release of the (+)-α-dihydrotetrabenazine from the capsule until the capsule reaches the stomach.

Alternatively, the capsule may be provided with an enteric coating if it is desired to delay release of the (+)-α-dihydrotetrabenazine until the capsule has reached the small intestine. Examples of such enteric coatings include methyl acrylate-methacrylic acid copolymers, methyl methacrylate-methacrylic acid copolymers, phthalates (such as cellulose acetate phthalate (CAP), hydroxypropyl methyl cellulose phthalate, polyvinyl acetate phthalate (PVAP)), cellulose succinates (such as cellulose acetate succinate and hydroxypropyl methyl cellulose acetate succinate) shellac, cellulose acetate trimellitate or sodium alginate.

The immediate release dosage form may release at least 70%, preferably at 80% or at least 90%, of the (+)-α-dihydrotetrabenazine content within a period of 4 hours, typically 3 hours, preferably 2 hours, more preferably within 1.5 hours, and especially within an hour (such as within 30 minutes), following oral administration of the formulation. In an embodiment, the (+)-α-dihydrotetrabenazine is provided in an immediate release dosage form which releases 80% of its (+)-α-dihydrotetrabenazine content within a period of 2 hours following oral administration.

The solid dosage forms (e.g.; tablets, capsules etc.) can be coated or un-coated, but typically have a coating, for example a protective film coating (e.g. a wax or varnish) or a release controlling coating. The coating (e.g. a Eudragit ^{™} type polymer) can be designed to release (+)-α-dihydrotetrabenazine at a desired location within the gastro-intestinal tract. Thus, the coating can be selected so as to degrade under certain pH conditions within the gastrointestinal tract, thereby selectively release the compound in the stomach or in the ileum or duodenum.

Based on the findings by the present inventors, it is envisaged that therapeutically VMAT2 blocking levels can be achieved for up to 12 hours following administration of (+)-α-dihydrotetrabenazine.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

Particular pharmaceutical compositions of the invention are compositions selected from:
- Sublingual compositions (e.g. sublingual tablets/strips, liquid drops or liquid sprays);
- Intranasal sprays;
- Pellets or tablets formulated to provide release kinetics corresponding to zero order release of the active compound;
- Pellets or tablets formulated to provide first fast release followed by constant rate release (zero order) of the active compound;
- Pellets or tablets formulated to provide a mixture of first order and zero order release of the active compound; and
- Pellets or tablets formulated to provide a combination of zero order and first order release of the active compound; and optionally a further order of release of the active compound selected from second, third and fourth orders of release and combinations thereof.

In any of the above Embodiments, the (+)-α-dihydrotetrabenazine may be administered to the patient using a sublingual or intranasal spray. The spray typically comprises the (+)-α-dihydrotetrabenazine in a solvent. The spray is suitably supplied to the patient in a bottle fitted with a mechanism for delivering the liquid in the bottle in the form of a spray.

Pellets and tablets formulated to provide release kinetics of the types defined above can be prepared according to methods well known the skilled person; for example as described in Remington's Pharmaceutical Sciences *(idem)* and "Remington - The Science and Practice of Pharmacy, 21st edition, 2006, ISBN 0-7817-4673-6.

The compounds of the inventions will generally be presented in unit dosage form and, as such, will typically contain sufficient compound to provide a desired level of biological activity. Such amounts are set out above.

The active compound will be administered to a subject (patient) in need thereof (for example a human or animal patient) in an amount sufficient to achieve the desired therapeutic effect.

### Brief Description of the Drawings

Figure 1 is a plot of % VMAT2 binding vs body weight after administration of doses of 7.5 mg, 15 mg and 22.5 mg of (+)-α-dihydrotetrabenazine to human subjects.
Figure 2 is a plot of % VMAT2 binding vs amounts of (+)-α-dihydrotetrabenazine administered to human subjects in mg/kg body weight.
Figure 3 shows the average total distance travelled by rats when treated with vehicle (with or without amphetamine induction) and (+)-α-dihydrotetrabenazine at doses of 0.5, 1, 1.5 and 2 mg/kg and risperidone at a dose of 1 mg/kg in amphetamine-induced rats, as described in Example 2, Study 1 below.
Figure 4 shows the average total stereotypic behaviour by rats when treated with vehicle (with or without amphetamine induction) and (+)-α-dihydrotetrabenazine at doses of 0.5, 1, 1.5 and 2 mg/kg and risperidone at a dose of 1 mg/kg in amphetamine-induced rats, as described in Example 2, Study 1 below.
Figure 5 shows the average total distance travelled by rats when treated with vehicle (with or without amphetamine induction) and (+)-α-dihydrotetrabenazine at doses of 0.1 mg/kg and 0.25 mg/kg and risperidone at a dose of 1 mg/kg in amphetamine-induced rats, as described in Example 2, Study 2 below.
Figure 6 shows the average total stereotypic behaviour by rats when treated with vehicle (with or without amphetamine induction) and (+)-α-dihydrotetrabenazine at doses of 0.1 mg/kg and 0.25 mg/kg and risperidone at a dose of 1 mg/kg in amphetamine-induced rats, as described in Example 2, Study 2 below.
Figure 7 shows the average total distance travelled by rats when treated with vehicle and (+)-α-dihydrotetrabenazine at a dose of 2.5 mg/kg or 5 mg/kg and risperidone at a dose of 1 mg/kg in rats without amphetamine induction, as described in Example 2, Study 3 below.
Figure 8 shows the average total stereotypic behaviour by rats when treated with vehicle and (+)-α-dihydrotetrabenazine at a dose of 2.5 mg/kg or 5 mg/kg and risperidone at a dose of 1 mg/kg in rats without amphetamine induction, as described in Example 2, Study 3 below.
Figure 9 shows the average total distance travelled by rats when treated with vehicle and (+)-α-dihydrotetrabenazine and Valbenazine each at a dose of 1 mg/kg or 1.5 mg/kg and risperidone at a dose of 1 mg/kg in amphetamine-induced rats, as described in Example 2, Study 4 below.
Figure 10 shows the average total stereotypic behaviour by rats when treated with vehicle and (+)-α-dihydrotetrabenazine and Valbenazine each at a dose of 1 mg/kg or 1.5 mg/kg and risperidone at a dose of 1 mg/kg in amphetamine-induced rats, as described in Example 2, Study 4 below.

### EXAMPLES

The following non-limiting examples illustrate the biological properties of (+)-alpha-dihydrotetrabenazine and its suitability in treating movement disorders.

### EXAMPLE 1A - (+)-Dihydrotetrabenazine plasma levels and VMAT2 binding levels over a period of up to 3 hours following administration of (+)-dihydrotetrabenazine

(+)-α-Dihydrotetrabenazine in defined amounts was administered by oral dosing (as a solution in apple juice) to six human volunteers. Blood samples were taken at 30, 60, 120 and 180 minutes after drug administration. At 60 minutes after drug administration, PET scans were initiated and these were stopped at 120 minutes after drug administration.

The experiment was carried out at dosages of 7.5 mg, 15 mg and 22.5 mg.

### RESULTS

Table 1A shows the plasma concentrations in nanogrammes/ml of (+)-α-dihydrotetrabenazine in six human subjects, 0.5, 1, 1.5, 2 and 3 hours after a dose of 7.5 mg, 15 mg and 22.5 mg. Table 2A shows the % VMAT2 blocking following administration of 7.5 mg, 15 mg and 22.5 mg of (+)-α-dihydrotetrabenazine in all six subjects.

**Table 1A**

| | | **Subject #** | | | | | |
|---|---|---|---|---|---|---|---|
| | | **1** | **2** | **3** | **4** | **5** | **6** |
| **Body Weight (kg)** | | 112 | 76 | 129 | 59 | 91 | 68 |
| **Dose (oral)** | **Time (h)** | | | | | | |
| 7.5 mg | | | | | | | |
| | 0.5 | BLQ | 0.531 | 0.216 | 8.43 | 6.68 | BLQ |
| | 1 | 0.94 | 13.7 | 4.35 | 15.0 | 9.8 | 3.77 |
| | 1.5 | 2.39 | 10.8 | 6.91 | 20.7 | 13.5 | 3.06 |
| | 2 | 2.44 | 14.0 | 5.03 | 17.6 | 10.7 | 4.33 |
| | 3 | 3.01 | 22.2 | 6.96 | 19.6 | 11.2 | 9.18 |
| 15 mg | | | | | | | |
| | 0.5 | 4.02 | 7.99 | 1.2 | 26.7 | 15.6 | 5.41 |
| | 1 | 11.1 | 22.8 | 14.3 | 53.8 | 34.2 | 10.6 |
| | 1.5 | 10.7 | 46.4 | 17.9 | 42.5 | 27.1 | 16.1 |
| | 2 | 10.2 | 35.7 | 12.0 | 53.3 | 31.8 | 17.4 |
| | 3 | 10.6 | 46.5 | 18.2 | 60.2 | 27.1 | 14.9 |
| | | | | | | | |
| 22.5 mg | | | | | | | |
| | 0.5 | 9.61 | 5.23 | 9.04 | ND | 55.3 | 5.58 |
| | 1 | 18.0 | 21.8 | 34.7 | ND | 45.1 | 8.01 |
| | 1.5 | 16.8 | 36.2 | 29.8 | ND | 46.2 | 5.87 |
| | 2 | 14.9 | 40.2 | 26.3 | ND | 41.9 | 12.5 |
| | 3 | 13.2 | 51.8 | 17.3 | ND | 42.5 | 18.7 |

| | | | | | | | |
|---|---|---|---|---|---|---|---|
| *BLQ* - *Below level of quantitation, ND* - *Not done* | | | | | | | |

**Table 2A**

| | | **Subject #** | | | | | |
|---|---|---|---|---|---|---|---|
| | | **1** | **2** | **3** | **4** | **5** | **6** |
| **Body Weight (kg)** | | 112 | 76 | 129 | 59 | 91 | 68 |
| **Dose** | 7.5 mg | 54 | 73 | 62 | 84 | 73 | 60 |
| **(oral)** | | | | | | | |
| | 15 mg | 73 | 83 | 69 | 89 | 79 | 76 |
| | 22.5 mg | 75 | 82 | 74 | ND | 82 | 62 |

Although in subjects with a lower body weight, higher (+)-α-dihydrotetrabenazine blood plasma concentrations were observed for a given dose, it can be seen that even in heavier individuals, at least 50% % VMAT2 blocking was observed at doses as low as 7.5 mg and, in lighter individuals, significantly higher % VMAT2 binding was. It was also observed that during the period of PET scanning, average plasma levels of less than 15 ng/ml gave rise to % VMAT2 binding of at least 50%.

The data demonstrate that very low doses of (+)-α-dihydrotetrabenazine resulting in plasma concentrations of less than 15 ng/ml can still give high levels of VMAT2 blocking.

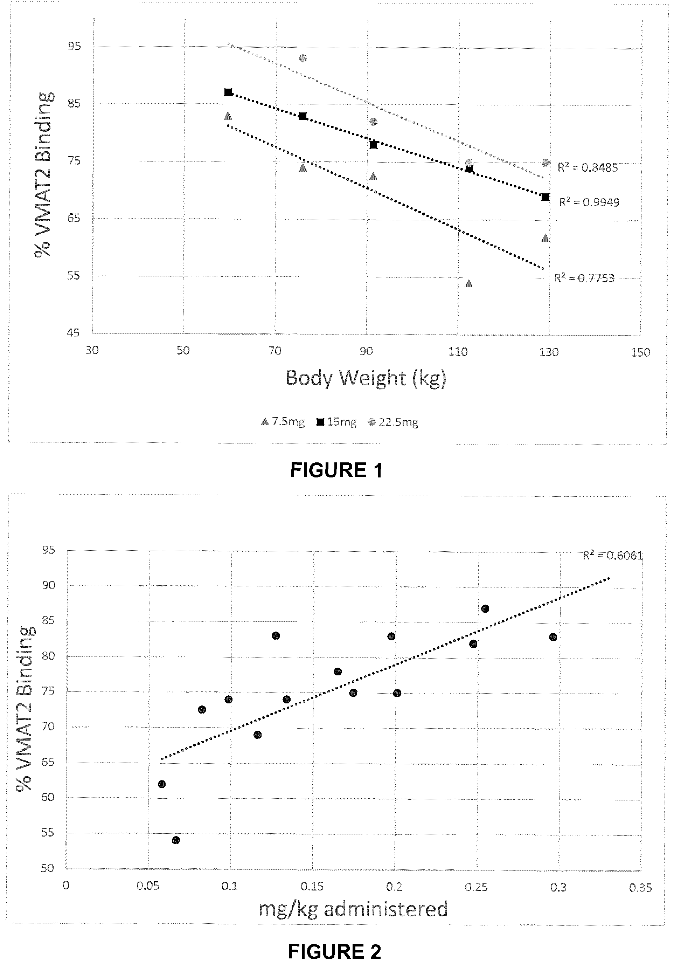

The data for subjects 1 to 5 were used to prepare plots (Figure 1) of % VMAT2 binding against body weight at the three dosage levels and plots (Figure 2) of % VMAt2 binding against the amount (in mg/kg body weight) of (+)-α-dihydrotetrabenazine administered. The data for subject 6, which are somewhat anomalous, were not included in Figure 1 or Figure 2.

Figure 1 shows the % VMAT2 binding against body weight for each dose of (+)-α-DHTBZ administered (7.5 mg, 15 mg and 22.5 mg) based on the data above. As can be seen, for a given dose there is a good correlation between body weight and %VMAT2 binding.

Figure 2 shows the % VMAT2 binding against the amount of (+)-α-DHTBZ per kg of the body weight of the subject based on the data above. Again it can be seen that there is a good correlation between amount of (+)-α-DHTBZ per weight of the subject (mg/kg value) and %VMAT2 binding.

Therefore, it is expected that the amount of (+)-α-DHTBZ that needs to be administered to provide a given VMAT2 binding level will depend greatly on the weight of the subject.

### EXAMPLE 1B - (+)-Dihydrotetrabenazine plasma levels and VMAT2 binding levels over a period of up to 7 hours following administration of (+)-dihydrotetrabenazine

(+)-α-Dihydrotetrabenazine in an amount of 7.5 mg was administered by oral dosing (dispersed in apple juice) to five human volunteers. Volunteers 1 and 2 were the same as volunteers 1 and 2 in Example 1A described above. Volunteers 3 to 5 did not partake in the study described in Example 1A. Blood sample were taken from all volunteers at 2, 4, 6 and 7 hours after drug administration. In addition, in all volunteers PET scans were initiated at 6 hours after drug administration and these were stopped at 7 hours after drug administration.

### RESULTS

Table 1B shows the plasma concentrations in nanogrammes/ml of (+)-α-dihydrotetrabenazine in six subjects up to 7 hours after a dose of 7.5 mg. Table 2B shows the % VMAT2 blocking in both the caudate and putamen regions of the striatum following administration of 7.5 mg of α-dihydrotetrabenazine in the same six subjects .

**Table 1B**

| | | **Subject #** | | | | | |
|---|---|---|---|---|---|---|---|
| | | **1** | **2** | **3** | **4** | **5** | **6** |
| **Body Weight (kg)** | | 112 | 76 | 76 | 95 | 99 | 107 |
| **Dose (oral)** | **Time (h)** | | | | | | |
| 7.5 mg | 1 | ND | ND | ND | 21.4 | 5.64 | 4.86 |
| | 2 | 8.7 | 11.7 | 12.8 | 13.9 | 7.59 | 6.3 |
| | 3 | ND | ND | ND | 10.6 | 5.84 | 6.69 |
| | 4 | 6.01 | 17.5 | 7.92 | 7.81 | 4.58 | 5.61 |
| | 5 | ND | ND | ND | 6.59 | 3.66 | 5.19 |
| | 6 | 4.54 | 13.4 | 5.57 | 4.92 | 3.35 | 4.44 |
| | 7 | 3.65 | 11.6 | 4.84 | 4.91 | 2.47 | 3.36 |

| | | | | | | | |
|---|---|---|---|---|---|---|---|
| *ND* - *Not done* | | | | | | | |

**Table 2B**

| | | **Subject #** | | | | | |
|---|---|---|---|---|---|---|---|
| | | **1** | **2** | **3** | **4** | **5** | **6** |
| **Body Weight (kg)** | **Dose (oral)** | 112 | 76 | 76 | 95 | 99 | 107 |
| %VMAT2 Occupancy Caudate | 7.5 mg | 38 | 65 | 41 | 34 | 36 | 44 |
| %VMAT2 Occupancy Putamen | | 39 | 63 | 42 | 32 | 36 | 49 |

The results show that despite blood plasma concentrations of (+)-α-dihydrotetrabenazine falling below 15ng/ml after 6 hours following administration of 7.5mg of (+)-α-dihydrotetrabenazine, the %VMAT2 blocking remains significant at greater than 30%.

By comparing the %VMAT2 blocking results for subjects 1 and 2 with the data provided in Example 1, it can be seen that the level of blocking after 6-7 hours following administration is reduced only by 30% and 11% (respectively for subjects 1 and 2) compared to the %VMAT2 blocking observed after 1-2 hours following administration.

No significant difference was observed between the %VMAT2 blocking levels in the caudate and putamen regions of the striatum for each subject.

Based on these findings it is expected that doses of 7.5mg or less of (+)-α-dihydrotetrabenazine can produce therapeutic levels of VMAT2 blocking for periods of in excess of 7 hours following administration.

### EXAMPLE 2 - Comparison of the effect of dihydrotetrabenazines and risperidone on amphetamine-induced hyperlocomotion

Dopaminergic models for Tourette's syndrome use systemic or focal administration of dopamine agonists such as amphetamine. After injection with amphetamine, a test animal expresses stereotypic behaviour. In particular, involvement of a dopaminergic system implicated in Tourette's syndrome wild type mice and rats can be stimulated with amphetamine and the resulting hyperactivity and stereotypies can be reversed with dopamine antagonists such as risperidone and haloperidol (Tourette's syndrome - Animal Models for Screening, Charles River Discovery Research Services, Finland).

Amphetamine produced a rise in extracellular concentrations of brain dopamine and concomitant behavioural manifestations in the rat and other species. At relatively low doses (1.2 ng/kg i.p.) amphetamine increases locomotor behaviour, ceases movement and gives way to a stationary posture accompanied by highly repetitive rapid head movements. This latter non-locomotor phase of stimulation is referred to as focused stereotypy. The stereotypy can last for over an hour and is usually followed by a period of locomotor stimulation (Schiorring 1971).

Administration of dopamine agonists (such as amphetamine) is known to induce behavioural stereotypies and sensorimotor gating disruption. Also, dopaminergic, cholinergic (TANs) and HDC models (subsequent to stress and/or amphetamine injection) are known to show an increase in stereotypic behaviours (Yaol et al 2016).

Amphetamine induced stereotype behaviour has also been evaluated as a model for the movement disorder condition, tardive dyskinesia (see Rubovitis et al (1972)).

The atypical antipsychotic drug risperidone is commonly used for the treatment of Tourette's syndrome and has been described (J. D. Walkup, A Guide to Tourette Syndrome Medications, Publ. 2008, The National Tourette Syndrome Association, Inc.) as being probably the best atypical antipsychotic for tic suppression with potentially less risk of motor side effects than haloperidol and fluphenazine.

Three studies were carried out to compare the effects of dihydrotetrabenazines and risperidone on amphetamine-induced and non-amphetamine-induced hyperlocomotion in rats, on the basis that, for the reasons given above, locomotor studies are useful models for Tourette's syndrome and other movement disorders.

### MATERIALS AND METHODS

### Equipment

Open field arena, Med Associates Inc.
Plastic syringes 1 ml, Terumo. Ref: SS-01T1
Animal feeding needle 15 G, Instech Solomon, Cat: 72-4446
Sartorius Mechatronics Scale A22101, Sartorius Weighting Technology, Germany Needle 27 G Terumo Myjector, 0,5 ml, Ref: 8300010463
Plastic syringes 3 ml, Soft-Ject, Ref: 8300005761
BD Microtainer K2EDTA tubes Ref: 365975
Matrix 0,75 ml, Alphanum Tubes, Thermo Scientific, Ref: 4274
Microplate Devices, Uniplate 24 wells, 10 ml, Ref: 734-1217
Thermo Electron Corp. Heraeus Fresco 17, refrigerated centrifuge

### Test Animals

All animal experiments were carried out according to the National Institute of Health (NIH) guidelines for the care and use of laboratory animals, and approved by the National Animal Experiment Board, Finland. Male CD (Charles River Laboratories, Germany) at weight range of 200-250 g (165-200 g upon arrival) were used for the experiments. Animals were housed at a standard temperature (22 ± 1°C) and in a light-controlled environment (lights on from 7 am to 8 pm) with ad libitum access to food and water.

### Methods

Locomotor activity of the rats was tested in open field arena. The open field test was performed during the rat light cycle and under a normal lighting evenly distributed to the test chambers. The paths of the rats were recorded by activity monitor (Med. Associates Inc.).

Dosing the vehicle, vehicle-amphetamine, (+)-α-DHTBZ or risperidone was done prior to LMA test. The rats were placed in the centre of the arena, and the path was recorded for 60 minutes.

### Endpoint, Blood Samples and Tissue Processing

Within 10 minutes from the end of the test animals were euthanized by an overdose of CO₂. The terminal blood sample was collected with cardiac puncture from all compound treated rats from each group excluding vehicle rats. 0.5 ml of blood was collected with syringe attached to 18 G needle and moved into precooled K2-EDTA microtubes. The EDTA microtube was inverted several times to mix up the EDTA and blood. Tubes were then immediately put on wet ice and centrifuged (Heraeus Fresco 17) within 10-15 minutes of collecting (9.6 x1000 G/ 10 x 1000 RPM, +4°C for 2 min), and 200 µl of plasma was collected in 96-tube plates (Matrix Technologies ScreenMates 0.75 ml Alphanumeric Round-Bottom Storage tubes, PP) on dry ice according to sample map.

After collection of blood the neck was dislocated at the base of the skull. Brain was collected and weighed. Brain weights were recorded and the brain was frozen on dry ice on the 24 well plate.

The plasma and brain samples were stored at -80°C until sent for analysis or destroyed.

### STUDY 1

The effects on stereotypic behaviour and the distance travelled in rats following administration of (+)-α-dihydrotetrabenazine dosed at 0.5 mg/kg to 2 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 0.5 mg/kg (t=0 min) and Amphetamine (t= 30 min)
- Group 4: 10 rats treated with (+)-α-DHTBZ 1 mg/kg (t=0 min) and Amphetamine (t= 30 min)
- Group 5: 10 rats treated with (+)-α-DHTBZ 1.5 mg/kg (t=0 min) and Amphetamine (t= 30 min)
- Group 6: 10 rats treated with (+)-α-DHTBZ 2 mg/kg (t=0 min) and Amphetamine (t= 30 min)
- Group 7: 10 rats treated with risperidone 1 mg/kg (t=0 min) and Amphetamine (t= 30 min)

### Results

### 1. Distance Travelled

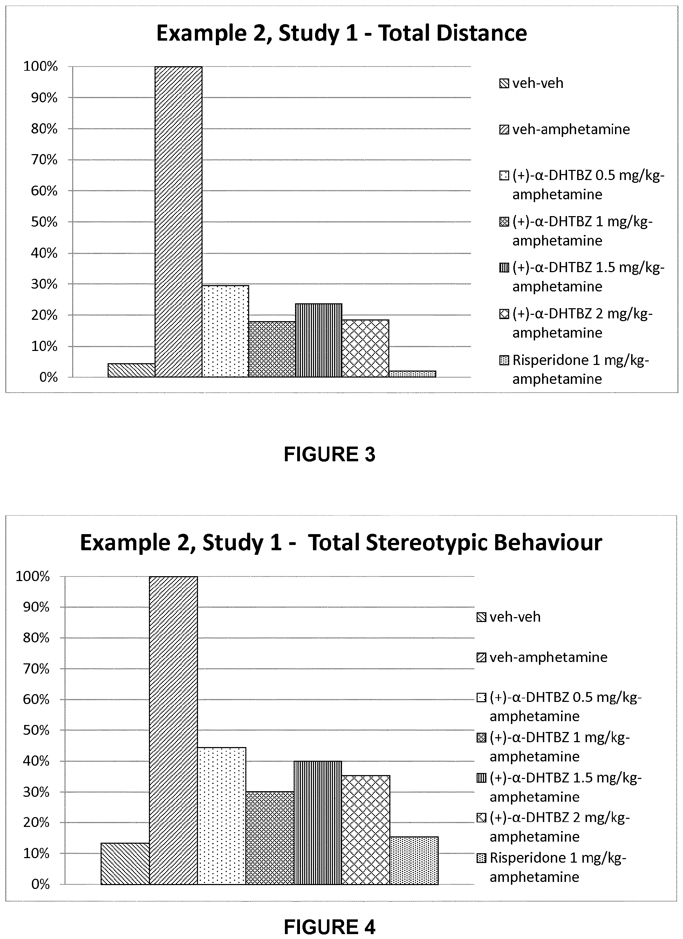

Rats dosed with either vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg or Risperidone 1 mg/kg were subjected to LMA testing first for 30 min and then for 60 minutes after vehicle or amphetamine challenge. Resulting locomotor activity was evaluated in 3 min bins and as a total over the testing period. The normalised total distance travelled over the testing time is presented in Figure 3.

When compared to the vehicle-vehicle group the vehicle-amphetamine was significantly different. When compared to vehicle-amphetamine group the vehicle-vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg and risperidone 1 mg/kg were significantly different.

### 2. Stereotypic Behaviour

Rats dosed with either vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 4.

When compared to the vehicle-vehicle group the vehicle-amphetamine, (+)-α-DHTBZ 0.5 mg/kg and (+)-α-DHTBZ 1.5 mg/kg were significantly different. When compared to vehicle-amphetamine group the vehicle-vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg and risperidone 1 mg/kg were significantly different.

### Conclusions

This study evaluated the effect of (+)-α-DHTBZ at doses 0.5 mg/kg, 1 mg/kg, 1.5 mg/kg and 2 mg/kg and risperidone at dose 1 mg/kg on amphetamine induced locomotor activity in male CD rats.

(+)-α-DHTBZ at all the tested doses and risperidone 1 mg/kg led to lower locomotor activity when compared to the vehicle-amphetamine group. (+)-α-DHTBZ at all the tested doses and risperidone 1 mg/kg led to reduced stereotypic behaviour when compared to the vehicle-amphetamine group. Both of the measured parameters suggest that (+)-α-DHTBZ has a sedative effect similar to risperidone.

### STUDY 2

The effects on stereotypic behaviour and the distance travelled in rats following administration of (+)-α-dihydrotetrabenazine dosed at 0.1 mg/kg to 0.25 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 0.1 mg/kg (t=0 min) and Amphetamine (t=30 min)
- Group 4: 10 rats treated with (+)-α-DHTBZ 0.25 mg/kg (t=0 min) and Amphetamine (t=30 min)
- Group 5: 10 rats treated with risperidone 1 mg/kg (t=0 min) and Amphetamine (t= 30 min)

### Results

### 1 Distance Travelled

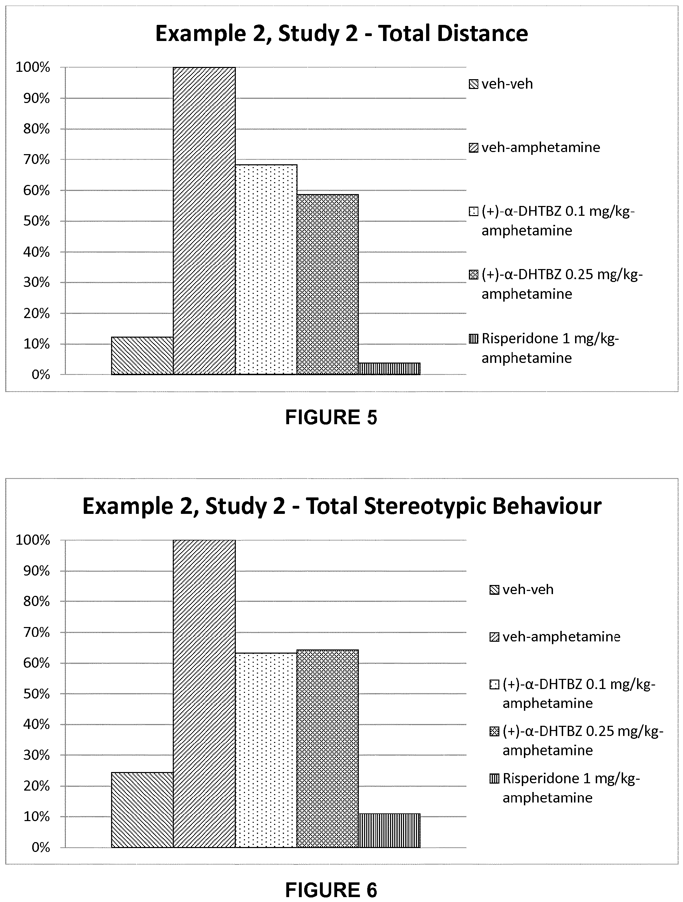

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting locomotor activity was evaluated in 3 min bins and as a total over the testing period. The normalised total distance travelled over the testing time is presented in Figure 5.

When compared to vehicle-amphetamine group the vehicle-vehicle, (+)-α-DHTBZ 0.25 mg/kg and risperidone 1 mg/kg were significantly different.

### 2 Stereotypic Behaviour

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 6.

When compared to vehicle-amphetamine group the vehicle-vehicle, (+)-α-DHTBZ 0.1 mg/kg, (+)-α-DHTBZ 0.25 mg/kg and risperidone 1 mg/kg were significantly different.

### Conclusions

This study evaluated the effect of (+)-α-DHTBZ at doses of 0.1 mg/kg and 0.25 mg/kg and risperidone at dose 1 mg/kg on amphetamine induced locomotor activity in male CD rats.

(+)-α-DHTBZ at 0.25 mg/kg and risperidone 1 mg/kg led to lower locomotor activity when compared to the vehicle-amphetamine group. (+)-α-DHTBZ at both the tested doses and risperidone 1 mg/kg led to reduced stereotypic behaviour when compared to the vehicle-amphetamine group.

### STUDY 3

The effects of (+)-α-dihydrotetrabenazine and risperidone on in non-amphetamine induced rats was studied. Animals were grouped as follows:
- Group 1: 10 rats treated with Vehicle
- Group 2: 10 rats treated with (+)-α-DHTBZ 2.5 mg/kg
- Group 3: 10 rats treated with (+)-α-DHTBZ 5 mg/kg
- Group 4: 10 rats treated with risperidone 1 mg/kg

### Results

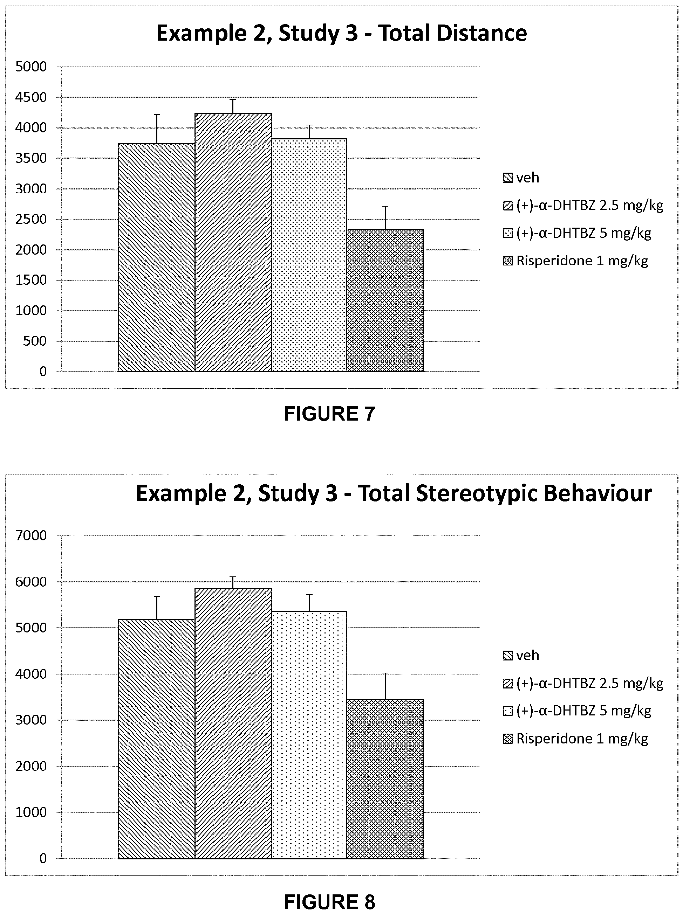

In non-induced rats, the total movement and stereotypic behaviour in rats treated with the vehicle were comparable to (+)-α-dihydrotetrabenazine (see Figures 7 and 8). However, rats treated with risperidone had reduced total movement and reduced total stereotypic behaviour.

### STUDY 4

The effects on stereotypic behaviour and the distance travelled in rats following administration of (+)-α-dihydrotetrabenazine and Valbenazine both dosed at 1 mg/kg and 1.5 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 1 mg/kg (t=0 min) and Amphetamine (t=30 min)
- Group 4: 10 rats treated with (+)-α-DHTBZ 1.5 mg/kg (t=0 min) and Amphetamine (t=30 min)
- Group 5: 10 rats treated with Valbenazine 1 mg/kg (t=0 min) and Amphetamine (t=30 min)
- Group 6: 10 rats treated with Valbenazine 1.5 mg/kg (t=0 min) and Amphetamine (t=30 min)
- Group 7: 10 rats treated with risperidone 1 mg/kg (t=0 min) and Amphetamine (t= 30 min)

### Results

### 1 Distance Travelled

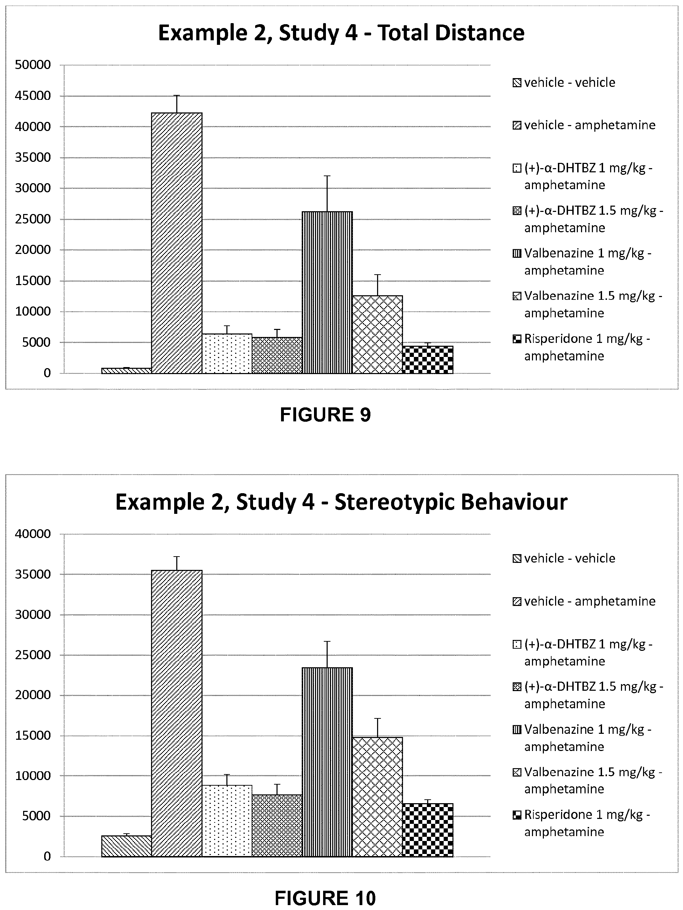

Rats dosed with either vehicle, (+)-α-DHTBZ, Valbenazine or Risperidone were subjected to LMA testing first for 30 min and then for 60 minutes after vehicle or amphetamine challenge. Resulting locomotor activity was evaluated in 3 min bins and as a total over the testing period. The total distance travelled over the testing time is presented in Figure 9.

When compared to vehicle-amphetamine group the vehicle-vehicle, (+)-α-DHTBZ (at 1 mg/kg and 1.5 mg/kg), Valbenazine (at 1 mg/kg and 1.5 mg/kg) and risperidone 1 mg/kg were significantly different.

### 2 Stereotypic Behaviour

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 10.

When compared to vehicle-amphetamine group the vehicle-vehicle, (+)-α-DHTBZ (at 1 mg/kg and 1.5 mg/kg), Valbenazine (at 1 mg/kg and 1.5 mg/kg) and risperidone 1 mg/kg were significantly different.

### Conclusions

This study evaluated the effect of (+)-α-DHTBZ and Valbenazine both at doses of 1 mg/kg and 1.5 mg/kg and risperidone at dose 1 mg/kg on amphetamine induced locomotor activity in male CD rats.

(+)-α-DHTBZ at 1 mg/kg and 1.5 mg/kg led to lower locomotor activity when compared to the vehicle-amphetamine group and the corresponding dose of Valbenazine. (+)-α-DHTBZ at both the tested doses led to reduced stereotypic behaviour when compared to the vehicle-amphetamine group and the corresponding dose of Valbenazine.

### Comments

Studies 1 and 2 in Example 2 show the effectiveness of doses of (+)-α-dihydrotetrabenazine as low as 0.1 mg/kg in reducing movement in amphetamine-induced rats. It is therefore expected that such low dose regimes may also be useful in treating hyperkinetic movement disorders in humans.

Study 3 in Example 2 suggests that following administration of low doses of (+)-α-dihydrotetrabenazine whereas abnormal movements of the type found in movement disorders will be reduced or suppressed by the drug, normal movements will not be. This is in contrast to risperidone, a well-used treatment for movement disorders, where the levels of both normal and abnormal movements can be reduced by administration of the drug.

Study 4 in Example 2 shows the increased effectiveness of (+)-α-dihydrotetrabenazine over Valbenazine.

### EXAMPLE 3

### PHARMACEUTICAL FORMULATIONS

### 3A. 7.5 mg Immediate Release Tablet

An immediate release tablet containing 7.5 mg of (+)-α-dihydrotetrabenazine is prepared by direct compression of the following composition.

| **Ingredient** | **Amount (mg)** | **% By Weight** |
|---|---|---|
| (+)-α-dihydrotetrabenazine | 7.5 | 8.33 |
| lactose | 42.4 | 47.11 |
| microcrystalline cellulose | 36.0 | 40.00 |
| crospovidone (crosslinked polyvinylpyrrolidone) | 3.5 | 3.89 |
| magnesium stearate | 0.6 | 0.67 |
| **Totals** | 90 | 100 |

### 3B. 5 mg Immediate Release Tablet

An immediate release tablet containing 7.5 mg of (+)-α-dihydrotetrabenazine is prepared by direct compression of the following composition.

| **Ingredient** | **Amount (mg)** | **% By Weight** |
|---|---|---|
| (+)-α-dihydrotetrabenazine | 5.0 | 5.56 |
| lactose | 44.9 | 49.89 |
| microcrystalline cellulose | 36.0 | 40.00 |
| crospovidone (crosslinked polyvinylpyrrolidone) | 3.5 | 3.89 |
| magnesium stearate | 0.6 | 0.67 |
| **Totals** | 90 | 100 |

### 3C. 2.5 mg Immediate Release Tablet

An immediate release tablet containing 7.5 mg of (+)-α-dihydrotetrabenazine is prepared by direct compression of the following composition.

| **Ingredient** | **Amount (mg)** | **% By Weight** |
|---|---|---|
| (+)-α-dihydrotetrabenazine | 2.5 | 5.56 |
| lactose | 22.45 | 49.89 |
| microcrystalline cellulose | 18.0 | 40.00 |
| crospovidone (crosslinked polyvinylpyrrolidone) | 1.75 | 3.89 |
| magnesium stearate | 0.3 | 0.67 |
| **Totals** | 45 | 100 |

### 3D. 5 mg Immediate Release Capsule

A capsule formulation is prepared by mixing 5 mg of (+)-α-dihydrotetrabenazine or an equimolar amount of a pharmaceutically acceptable salt thereof with 75 mg lactose and filling the resulting mixture into standard opaque hard gelatin capsules.

## Claims

1. A VMAT2 blocking compound for use in a method of treating a subject suffering from a movement disorder; which method comprises administering to the subject a therapeutically effective amount of the VMAT2 blocking compound so as to provide in the subject a therapeutically effective level of VMAT2 blocking; said VMAT2 blocking being maintained such that there is at least 30% VMAT2 blocking after a period of 6.5 hours from administration of the VMAT2 blocking compound; wherein:
- the VMAT2 blocking compound is (+)-α-dihydrotetrabenazine or a pharmaceutically acceptable salt thereof;
- the administration of the therapeutically effective amount of the VMAT2 blocking compound takes place within an initial time window of 30 minutes, and no further administration of the VMAT2 blocking compound takes place within the period of 6.5 hours;
- the said therapeutically effective amount is no more than 8 mg of (+)-α-dihydrotetrabenazine free base or an equimolar amount of a pharmaceutically acceptable salt thereof;
and wherein the VMAT2 blocking compound is presented in an immediate release formulation.

2. A VMAT2 blocking compound for use according to claim 1 wherein the single time window is no more than 10 minutes.

3. A VMAT2 blocking compound for use according to claim 1 wherein the single time window is no more than 5 minutes.

4. A VMAT2 blocking compound for use according to claim 1 wherein the compound is administered in a single dose.

5. A VMAT2 blocking compound for use according to any one of claims 1 to 4 wherein the therapeutically effective amount is in the range from 0.5 mg to 7.5 mg of (+)-α-dihydrotetrabenazine free base or an equimolar amount of a pharmaceutically acceptable salt thereof.

6. A VMAT2 blocking compound for use according to any one of claims 1 to 5 wherein the (+)-α-dihydrotetrabenazine is administered in the form of a tablet comprising (+)-α-dihydrotetrabenazine or a pharmaceutically acceptable salt thereof and a disintegrant.

7. A VMAT2 blocking compound for use according to any one of claims 1 to 5 wherein the (+)-α-dihydrotetrabenazine or a pharmaceutically acceptable salt thereof is administered in the form of a tablet formed by direct compression.

8. A VMAT2 blocking compound for use according to any one of claims 1 to 5 wherein the (+)-α-dihydrotetrabenazine or a pharmaceutically acceptable salt thereof is administered in the form of a chewable dosage form such as a chewable tablet, lozenge or gummie.

9. A VMAT2 blocking compound for use according to any one of claims 1 to 5 wherein the (+)-α-dihydrotetrabenazine or a pharmaceutically acceptable salt thereof is administered in the form of:
• an effervescent tablet containing an effervescent couple;
• a melt;
• a solution;
• a syrup;
• a suspension;
• a sublingual tablet, wafer or spray; or
• a capsule.

10. A VMAT2 blocking compound for use according to any one of claims 1 to 5 wherein the capsule is a hard-shelled (e.g. hard gelatin) capsule.

11. A VMAT2 blocking compound for use according to any one of claims 1 to 5 wherein the capsule is a soft-shelled (e.g. soft gelatin) capsule.

12. A VMAT2 blocking compound for use according to any one of claims 1 to 5 wherein the (+)-α-dihydrotetrabenazine or a pharmaceutically acceptable salt thereof is administered in the form of a powder.

13. A VMAT2 blocking compound for use according to any one of claims 1 to 5 wherein the (+)-α-dihydrotetrabenazine or a pharmaceutically acceptable salt thereof is administered in the form of an intranasal spray.

14. A VMAT2 blocking compound for use according to any one of claims 1 to 9, wherein when the (+)-α-dihydrotetrabenazine or a pharmaceutically acceptable salt thereof is administered in a solid form the (+)-α-dihydrotetrabenazine or a pharmaceutically acceptable salt thereof is in the form of particles having a D₉₀ value of less than 250 µm, more usually less than 250 µm, for example in the range from 40 to 150 µm as determined by laser diffraction.

15. A VMAT2 blocking compound for use according to any one of claims 1 to 14 wherein the movement disorder is selected from tardive dyskinesia, Tourette's syndrome and Huntington's disease.

## Patentansprüche

1. VMAT2-blockierende Verbindung zur Verwendung in einem Verfahren zum Behandeln eines Subjekts, das an einer Bewegungsstörung leidet; wobei das Verfahren Verabreichen einer therapeutisch wirksamen Menge der VMAT2-blockierenden Verbindung an das Subjekt umfasst, um in dem Subjekt einen therapeutisch wirksamen Grad an VMAT2-Blockierung bereitzustellen; wobei die VMAT2-Blockierung so aufrechterhalten wird, dass nach einem Zeitraum von 6,5 Stunden nach der Verabreichung der VMAT2-blockierenden Verbindung eine VMAT2-Blockierung von mindestens 30 % vorliegt, wobei:
- die VMAT2-blockierende Verbindung (+)-α-Dihydrotetrabenazin oder ein pharmazeutisch unbedenkliches Salz davon ist;
- die Verabreichung der therapeutisch wirksamen Menge der VMAT2-blockierenden Verbindung innerhalb eines anfänglichen Zeitfensters von 30 Minuten erfolgt und innerhalb des Zeitraums von 6,5 Stunden keine weitere Verabreichung der VMAT2-blockierenden Verbindung erfolgt;
- die therapeutisch wirksame Menge nicht mehr als 8 mg der freien Base von (+)-α-Dihydrotetrabenazin oder einer äquimolaren Menge eines pharmazeutisch unbedenklichen Salzes davon beträgt;
und wobei die VMAT2-blockierende Verbindung in einer Formulierung mit sofortiger Freisetzung vorliegt.

2. VMAT2-blockierende Verbindung zur Verwendung nach Anspruch 1, wobei das einzelne Zeitfenster nicht mehr als 10 Minuten beträgt.

3. VMAT2-blockierende Verbindung zur Verwendung nach Anspruch 1, wobei das einzelne Zeitfenster nicht mehr als 5 Minuten beträgt.

4. VMAT2-blockierende Verbindung zur Verwendung nach Anspruch 1, wobei die Verbindung in einer Einzeldosis verabreicht wird.

5. VMAT2-blockierende Verbindung zur Verwendung nach einem der Ansprüche 1 bis 4, wobei die therapeutisch wirksame Menge im Bereich von 0,5 mg bis 7,5 mg der freien Base von (+)-α-Dihydrotetrabenazin oder einer äquimolaren Menge eines pharmazeutisch unbedenklichen Salzes davon liegt.

6. VMAT2-blockierende Verbindung zur Verwendung nach einem der Ansprüche 1 bis 5, wobei das (+)-α-Dihydrotetrabenazin in Form einer Tablette verabreicht wird, die (+)-α-Dihydrotetrabenazin oder ein pharmazeutisch unbedenkliches Salz davon und ein Sprengmittel umfasst.

7. VMAT2-blockierende Verbindung zur Verwendung nach einem der Ansprüche 1 bis 5, wobei das (+)-α-Dihydrotetrabenazin oder ein pharmazeutisch unbedenkliches Salz davon in Form einer durch Direktverpressung gebildeten Tablette verabreicht wird.

8. VMAT2-blockierende Verbindung zur Verwendung nach einem der Ansprüche 1 bis 5, wobei das (+)-α-Dihydrotetrabenazin oder ein pharmazeutisch unbedenkliches Salz davon in Form einer kaubaren Dosierungsform wie einer Kautablette, einer Lutschpastille oder einem Weichgummi verabreicht wird.

9. VMAT2-blockierende Verbindung zur Verwendung nach einem der Ansprüche 1 bis 5, wobei das (+)-α-Dihydrotetrabenazin oder ein pharmazeutisch unbedenkliches Salz davon in der folgenden Form verabreicht wird:
• einer Brausetablette, die eine Brausemischung enthält;
• einer Schmelztablette;
• einer Lösung;
• eines Sirups;
• einer Suspension;
• einer sublingualen Tablette, eines Plättchens oder Sprays; oder
• einer Kapsel.

10. VMAT2-blockierende Verbindung zur Verwendung nach einem der Ansprüche 1 bis 5, wobei die Kapsel eine Hartkapsel (z. B. Hartgelatinekapsel) ist.

11. VMAT2-blockierende Verbindung zur Verwendung nach einem der Ansprüche 1 bis 5, wobei die Kapsel eine Weichkapsel (z. B. Weichgelatinekapsel) ist.

12. VMAT2-blockierende Verbindung zur Verwendung nach einem der Ansprüche 1 bis 5, wobei das (+)-α-Dihydrotetrabenazin oder ein pharmazeutisch unbedenkliches Salz davon in Form eines Pulvers verabreicht wird.

13. VMAT2-blockierende Verbindung zur Verwendung nach einem der Ansprüche 1 bis 5, wobei das (+)-α-Dihydrotetrabenazin oder ein pharmazeutisch unbedenkliches Salz davon in Form eines intranasalen Sprays verabreicht wird.

14. VMAT2-blockierende Verbindung zur Verwendung nach einem der Ansprüche 1 bis 9, wobei, wenn das (+)-α-Dihydrotetrabenazin oder ein pharmazeutisch unbedenkliches Salz davon in fester Form verabreicht wird, das (+)-α-Dihydrotetrabenazin oder ein pharmazeutisch unbedenkliches Salz davon in Form von Partikeln vorliegt, die einen D₉₀-Wert von weniger als 250 µm aufweisen, besonders üblich weniger als 250 µm, beispielsweise im Bereich von 40 bis 150 µm, bestimmt durch Laserbeugung.

15. VMAT2-blockierende Verbindung zur Verwendung nach einem der Ansprüche 1 bis 14, wobei die Bewegungsstörung aus Spätdyskinesie, Tourette-Syndrom und Chorea Huntington ausgewählt ist.

## Revendications

1. Composé de blocage de la VMAT2 destiné à être utilisé dans une méthode de traitement d'un sujet souffrant d'un trouble du mouvement ; laquelle méthode comprend l'administration au sujet d'une quantité thérapeutiquement efficace du composé de blocage de la VMAT2 de manière à assurer chez le sujet un niveau thérapeutiquement efficace de blocage de la VMAT2 ; ledit blocage de la VMAT2 étant maintenu de sorte qu'il existe un blocage d'au moins 30 % de la VMAT2 après une période de 6,5 heures à partir de l'administration du composé de blocage de la VMAT2 ;
- ledit : composé de blocage de la VMAT2 étant la (+)-α-dihydrotétrabénazine ou un sel pharmaceutiquement acceptable de celle-ci ;
- ladite administration de la quantité thérapeutiquement efficace du composé de blocage de la VMAT2 ayant lieu dans une fenêtre temporelle initiale de 30 minutes, et aucune administration supplémentaire du composé de blocage de la VMAT2 n'ayant lieu dans la période de 6,5 heures ;
- ladite quantité thérapeutiquement efficace n'étant pas supérieure à 8 mg de base libre de (+)-α-dihydrotétrabénazine ou d'une quantité équimolaire d'un sel pharmaceutiquement acceptable de celle-ci ;
et ledit composé de blocage de la VMAT2 se présentant dans une formulation à libération immédiate.

2. Composé de blocage de la VMAT2 destiné à être utilisé selon la revendication 1, ladite fenêtre temporelle unique n'étant pas supérieure à 10 minutes.

3. Composé de blocage de la VMAT2 destiné à être utilisé selon la revendication 1, ladite fenêtre temporelle unique n'étant pas supérieure à 5 minutes.

4. Composé de blocage de la VMAT2 destiné à être utilisé selon la revendication 1, ledit composé étant administré en une dose unique.

5. Composé de blocage de la VMAT2 destiné à être utilisé selon l'une quelconque des revendications 1 à 4, ladite quantité thérapeutiquement efficace se trouvant dans la plage de 0,5 mg à 7,5 mg de base libre de (+)-α-dihydrotétrabénazine ou d'une quantité équimolaire d'un sel pharmaceutiquement acceptable de celle-ci.

6. Composé de blocage de la VMAT2 destiné à être utilisé selon l'une quelconque des revendications 1 à 5, ladite (+)-α-dihydrotétrabénazine étant administrée sous la forme d'un comprimé comprenant de la (+)-α-dihydrotétrabénazine ou un sel pharmaceutiquement acceptable de celle-ci et un délitant.

7. Composé de blocage de la VMAT2 destiné à être utilisé selon l'une quelconque des revendications 1 à 5, ladite (+)-α-dihydrotétrabénazine ou un sel pharmaceutiquement acceptable de celle-ci étant administré sous la forme d'un comprimé formé par compression directe.

8. Composé de blocage de la VMAT2 destiné à être utilisé selon l'une quelconque des revendications 1 à 5, ladite (+)-α-dihydrotétrabénazine ou un sel pharmaceutiquement acceptable de celle-ci étant administré sous la forme d'une forme posologique masticable telle qu'un comprimé, une pastille ou une gomme masticable.

9. Composé de blocage de la VMAT2 destiné à être utilisé selon l'une quelconque des revendications 1 à 5, ladite (+)-α-dihydrotétrabénazine ou un sel pharmaceutiquement acceptable de celle-ci étant administré sous la forme de :
• un comprimé effervescent contenant un couple effervescent ;
• un mélange ;
• une solution ;
• un sirop ;
• une suspension ;
• un comprimé sublingual, un cachet ou un spray ; ou
• une gélule.

10. Composé de blocage de la VMAT2 destiné à être utilisé selon l'une quelconque des revendications 1 à 5, ladite gélule étant une gélule à enveloppe dure (par ex. une gélatine dure).

11. Composé de blocage de la VMAT2 destiné à être utilisé selon l'une quelconque des revendications 1 à 5, ladite gélule étant une gélule à enveloppe souple (par ex. une gélatine souple).

12. Composé de blocage de la VMAT2 destiné à être utilisé selon l'une quelconque des revendications 1 à 5, ladite (+)-α-dihydrotétrabénazine ou un sel pharmaceutiquement acceptable de celle-ci étant administré sous la forme d'une poudre.

13. Composé de blocage de la VMAT2 destiné à être utilisé selon l'une quelconque des revendications 1 à 5, ladite (+)-α-dihydrotétrabénazine ou un sel pharmaceutiquement acceptable de celle-ci étant administré sous la forme d'un spray intranasal.

14. Composé de blocage de la VMAT2 destiné à être utilisé selon l'une quelconque des revendications 1 à 9, lorsque ladite (+)-α-dihydrotétrabénazine ou un sel pharmaceutiquement acceptable de celle-ci est administré sous la forme d'un solide, ladite (+)-α-dihydrotétrabénazine ou un sel pharmaceutiquement acceptable de celle-ci étant sous la forme de particules présentant une valeur D₉₀ inférieure à 250 µm, plus généralement inférieure à 250 µm, par exemple dans la plage allant de 40 à 150 µm telle que déterminée par diffraction laser.

15. Composé de blocage de la VMAT2 destiné à être utilisé selon l'une quelconque des revendications 1 à 14, ledit trouble du mouvement étant choisi parmi la dyskinésie tardive, le syndrome de Tourette et la maladie de Huntington.
